# EUROPÄISCHE PATENTSCHRIFT

(11) **EP 0 934 252 B1**
(45) Veröffentlichungstag und Bekanntmachung des Hinweises auf die Patenterteilung: **26.09.2001**
(21) Anmeldenummer: 97910413.0
(22) Anmeldetag: 06.10.1997
(51) Int. Cl.: C07C 235/78, A01N 37/36

(54) **AMID-DERIVATE UND IHRE VERWENDUNG ALS SCHÄDLINGSBEKÄMPFUNGSMITTEL**
AMIDE DERIVATIVES AND THEIR USE AS PESTICIDES
DERIVES D'AMIDE ET LEUR UTILISATION COMME PARASITICIDES

(30) Priorität: 17.10.1996 DE 19642863
(43) Veröffentlichungstag der Anmeldung: 11.08.1999
(73) Patentinhaber: BAYER AG, 51368 Leverkusen (DE)
(72) Erfinder: SEITZ, Thomas, D-40764 Langenfeld (DE); STENZEL, Klaus, D-40595 Düsseldorf (DE)
(86) Internationale Anmeldenummer: EP9705474
(87) Internationale Veröffentlichungsnummer: WO9817630

(56) Entgegenhaltungen:
- DE-A- 4 319 887
- US-A- 5 556 873
- DATABASE WPI Week 9349 Derwent Publications Ltd., London, GB; AN 93-392623 XP002053030 "Prodn. of N,N-dialkyl mandelamide(s) used as insect repellents - by reacting 2,2-dimethyl-5-phenyl 1,3-dioxolanone and dialkylamine" & JP 05 294 911 A (YAMAKAWA YAKUHIN KOGYO) , 9.November 1993

## Beschreibung

Die Erfindung betrifft neue Amide, ein Verfahren zu ihrer Herstellung und ihre Verwendung als Schädlingsbekämpfungsmittel.

In DE-A1-43 19887 werden Arylacetamide, Verfahren zu ihre Herstellung und ihre Verwendung als Fungizide beschreiben.

Es wurden neue Verbindungen der allgemeinen Formel (I) gefunden, 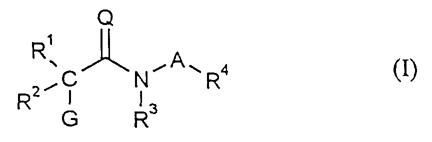 in welcher
- A: für eine Einfachbindung oder gegebenenfalls substituiertes Alkylen steht,
- Q: für Sauerstoff oder Schwefel steht,
- R¹: für Aryl steht, an das ein Cycloalkylring ankondensiert ist, wobei sowohl der Arylteil als auch der Cycloalkylteil gegebenenfalls weitere Substituenten trägt, oder
- R¹: für gegebenenfalls substituiertes, am Benzolring verknüpftes, Benzoheterocyclyl mit einem, zwei oder drei Heteroatomen steht, oder
- R¹: für einen Tricyclus 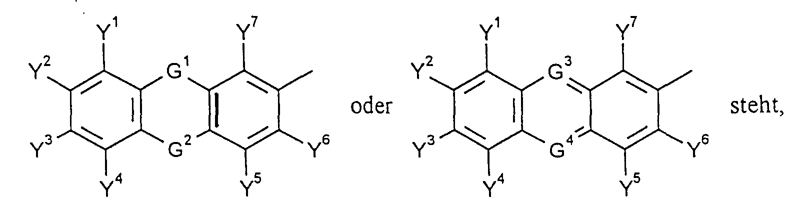 worin
G¹ und G² unabhängig voneinander für eine Einfachbindung, Alkandiyl, Alkendiyl, Sauerstoff, Schwefel, -NH-, -N(Alkyl)- oder Carbonyl stehen,
G³ und G⁴ unabhängig voneinander für Stickstoff oder eine Gruppierung 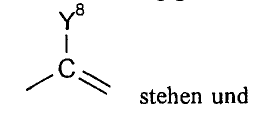
Y¹, Y², Y³, Y⁴, Y⁵, Y⁶, Y⁷ und Y⁸ unabhängig voneinander für Wasserstoff, Halogen, Cyano, Nitro, jeweils gegebenenfalls substituiertes Alkyl, Alkoxy, Alkylamino, Dialkylamino, Alkylthio, Alkylsulfinyl, Alkylsulfonyl oder Cycloalkyl stehen,
- R²: für Hydroxy oder Alkoxy steht,
- G: für Wasserstoff oder gemeinsam mit R² für ein zweifach gebundenes Sauerstoffatom steht,
- R³: für Wasserstoff oder jeweils gegebenenfalls substituiertes Alkyl, Alkenyl, Alkinyl oder Cycloalkyl steht,
- R⁴: für jeweils gegebenenfalls substituiertes Cycloalkyl, Cycloalkenyl, Aryl oder Heterocyclyl steht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält der Ring mehrere Sauerstoffatome, stehen diese nicht benachbart.

Ringförmige Verbindungen bilden gegebenenfalls mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Benzoheterocyclyl steht für einen heterocyclischen Ring, an den ein Benzolring ankondensiert ist.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Cycloalkenyl steht für carbocyclische, ringförmige Verbindungen, die mindestens eine Doppelbindung enthalten und gegebenenfalls mit weiteren carbocyclischen, ankondensierten oder überbrückten Ringen ein polycyclisches Ringsystem bilden.

Benzoheterocyclyl steht für einen heterocyclischen Ring, an den ein Benzolring ankondensiert ist.

Schließlich wurde gefunden, daß die neuen Amide der allgemeinen Formel (I) sehr starke fungizide Wirkung zeigen.

Die erfindungsgemäßen Verbindungen liegen gegebenenfalls als Mischungen verschiedener möglicher isomerer Formen, insbesondere von Stereoisomeren, wie z.B. E- und Z-, threo- und erythro-, sowie optischen Isomeren vor. Es werden sowohl die E- als auch die Z-Isomeren, wie auch die threo- und erythro-, sowie die optischen Isomeren sowie beliebige Mischungen dieser Isomeren beschrieben und beansprucht.

Gegenstand der Erfindung sind vorzugsweise Verbindungen der Formel (I), in welcher
- A: für eine Einfachbindung oder für gegebenenfalls einfach oder mehrfach, gleich oder verschieden substituiertes Alkylen mit 1 bis 6 Kohlenstoffatomen steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
   sowie jeweils gegebenenfalls einfach oder mehrfach, gleich oder verschieden durch

Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl,
   - Q: für Sauerstoff oder Schwefel steht,
   - R¹: für Phenyl oder Naphthyl steht, an das ein Cycloalkylring mit 3 bis 10 Ringgliedern ankondensiert ist, wobei der Cycloalkylteil gegebenenfalls durch 1 bis 4 Alkylketten mit jeweils 1 - 4 Kohlenstoffatomen substituiert ist und der Phenyl- bzw. Naphthylteil gegebenenfalls diejenigen Substituenten trägt, die für Y¹ bis Y⁸ genannt sind, oder
   - R¹: für gegebenenfalls einfach oder mehrfach, gleich oder verschieden substituiertes, am Benzolring verknüpftes, Benzoheterocyclyl mit 3 bis 12 Ringgliedern im Heterocyclylteil und einem, zwei oder drei Heteroatomen steht, wobei die möglichen Substituenten diejenigen sind, die für Y¹ bis Y⁸ genannt sind, oder
   - R¹: für einen Tricyclus 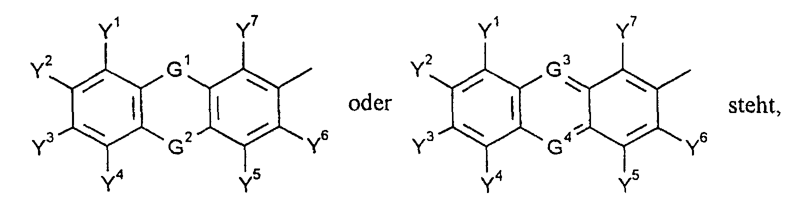
worin
- G¹ und G²: unabhängig voneinander für eine Einfachbindung, Alkandiyl, Alkendiyl, Sauerstoff, Schwefel, -NH-, -N(Alkyl)- oder Carbonyl stehen,
- G³ und G⁴: unabhängig voneinander für Stickstoff oder eine Gruppierung 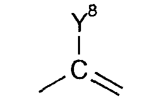 stehen und
- Y¹, Y², Y³, Y⁴, Y⁵, Y⁶, Y⁷ und Y⁸: unabhängig voneinander für Wasserstoff, Halogen, Cyano, Nitro;
Alkyl, Alkoxy, Alkylamino, Dialkylamino, Alkylthio, Alkylsulfinyl oder Alkylsulfonyl mit jeweils 1 bis 8 Kohlenstoffatomen in den einzelnen Alkylteilen;
Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen oder
Cycloalkyl mit 3 bis 6 Kohlenstoffatomen stehen,
- R²: für Hydroxy oder Alkoxy mit 1 bis 4 Kohlenstoffatomen steht,
- G: für Wasserstoff oder gemeinsam mit R² für ein zweifach gebundenes Sauerstoffatom steht,
- R³: für Wasserstoff oder Alkyl mit 1 bis 4 Kohlenstoffatomen steht,
- R⁴: für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten vorzugsweise aus der nachstehenden Aufzählung ausgewählt sind:

Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
   sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
   und/oder geradkettiges oder verzweigtes Halogenalkyl mit 1 bis 4 Kohlenstoffatomen und 1 bis 9 gleichen oder verschiedenen Halogenatomen
   und/oder geradkettiges oder verzweigtes Alkoxy oder Alkylthio mit 1 bis 4 Kohlenstoffatomen
   und/oder geradkettiges oder verzweigtes Halogenalkoxy oder Halogenalkylthio mit 1 bis 4 Kohlenstoffatomen und 1 bis 9 gleichen oder verschiedenen Halogenatomen
   und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
   substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

Die Erfindung betrifft insbesondere Verbindungen der Formel (I), in welcher
- A: für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3-oder 2,2-Propylen, 1,1-, 1,2-, 1,3-, 1,4-, 2,2-, 2,3-Butylen oder 1,1-, 1,2-oder 1,3-(2-Methyl-propylen) steht,
- Q: für Sauerstoff oder Schwefel steht,
- R¹: für Phenyl oder Naphthyl steht, an das ein Cyclopropyl-, Cyclobutyl-, Cyclopentyl-, Cyclohexyl-, Cycloheptyl- Cyclooctyl- oder Cyclononylring ankondensiert ist, wobei der Cycloalkylteil gegebenenfalls durch einfach bis vierfach durch Methyl, Ethyl, n- oder i-Propyl, n-, i-, s- oder t-Butyl substituiert ist und der Phenyl- bzw. Naphthylteil gegebenenfalls diejenigen Substituenten trägt, die für Y¹ bis Y⁸ genannt sind, oder
- R¹: für eine der nachstehenden Gruppierungen 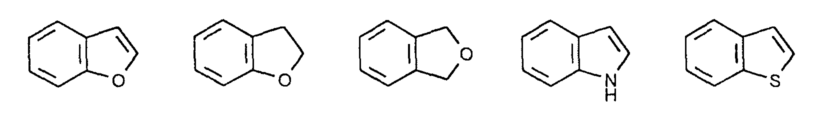 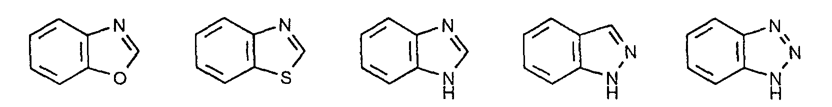 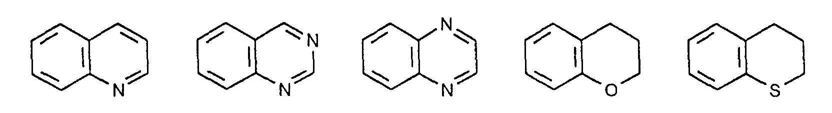 steht, die jeweils über ein Kohlenstoffatom des jeweiligen Benzolringes verbunden sind und die jeweils gegebenenfalls einfach bis dreifach, gleich oder verschieden durch diejenigen Substituenten substituiert sein können, die für Y¹ bis Y⁸ genannt sind, oder
- R¹: für einen Tricyclus 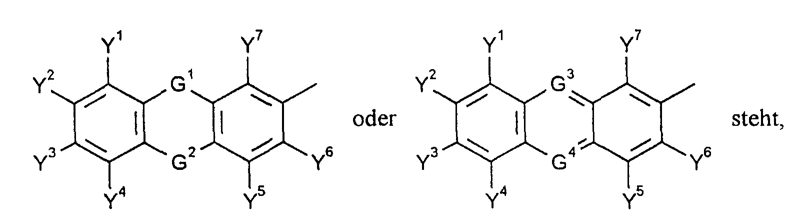 worin
G¹ und G² unabhängig voneinander für eine Einfachbindung, Methandiyl, Ethandiyl, Propandiyl, Ethendiyl, Sauerstoff, Schwefel, -NH-, -N(CH₃)- oder Carbonyl stehen und
G³ und G⁴ unabhängig voneinander für Stickstoff oder eine Gruppierung 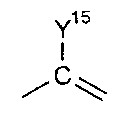 stehen und
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n-oder i-Propylamino, Dimethylamino, Diethylamino, Cyclopropyl, Cyclobutyl, Cyclopentyl oder Cyclohexyl stehen,
- R²: für Hydroxy, Methoxy oder Ethoxy steht,
- G: für Wasserstoff oder gemeinsam mit R² für ein zweifach gebundenes Sauerstoffatom steht,
- R³: für Wasserstoff oder für Methyl oder Ethyl steht,
- R⁴: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl,
Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Besonders bevorzugt sind Verbindungen der Formel (I), in welcher
- A: für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methylpropylen) steht,
- Q: für Sauerstoff oder Schwefel steht,
- R¹: für eine Gruppierung 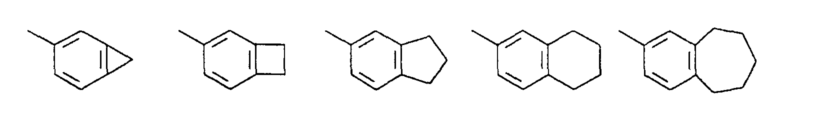 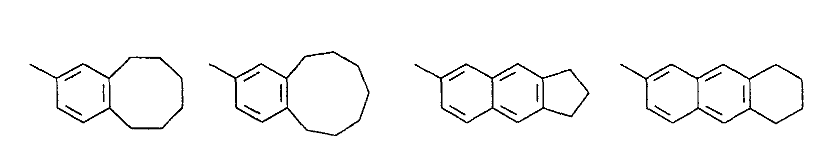 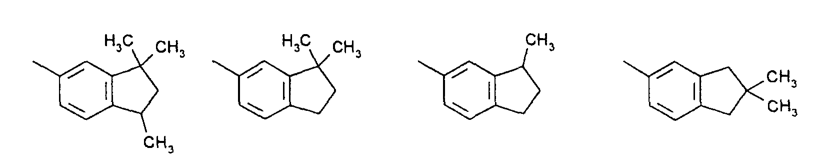 steht, der am Phenyl- bzw. Naphthylteil gegebenenfalls diejenigen Substituenten trägt, die für Y¹ bis Y⁸ genannt sind, oder
- R¹: für eine der nachstehenden Gruppierungen 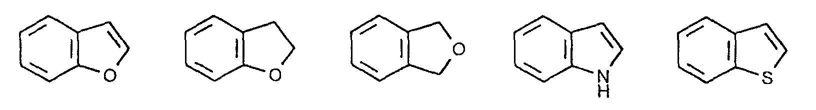 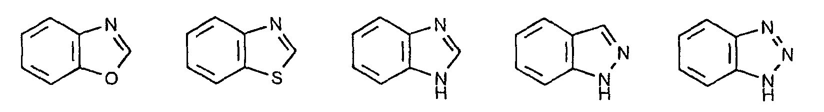 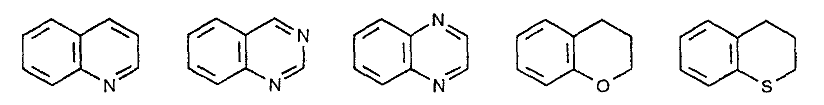 steht, die jeweils über ein Kohlenstoffatom des jeweiligen Benzolringes verbunden sind und die jeweils gegebenenfalls einfach bis dreifach, gleich oder verschieden durch diejenigen Substituenten substituiert sein können, die für Y¹ bis Y⁸ genannt sind, oder
- R¹: für einen Tricyclus 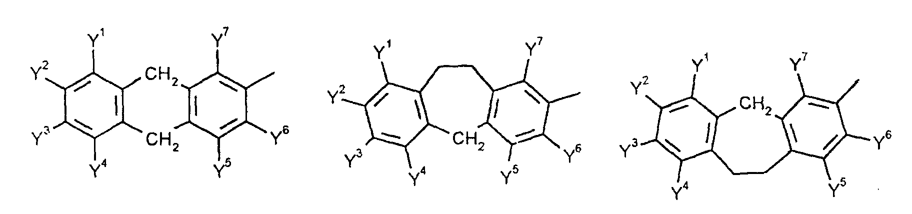 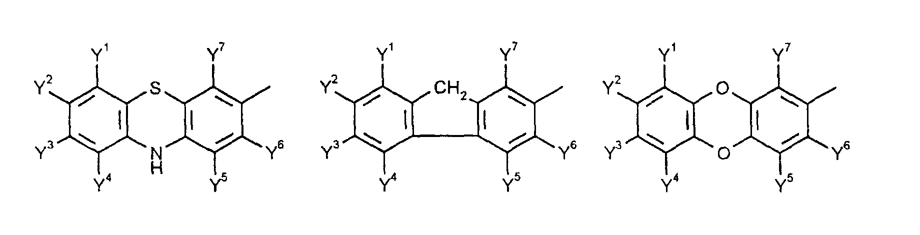 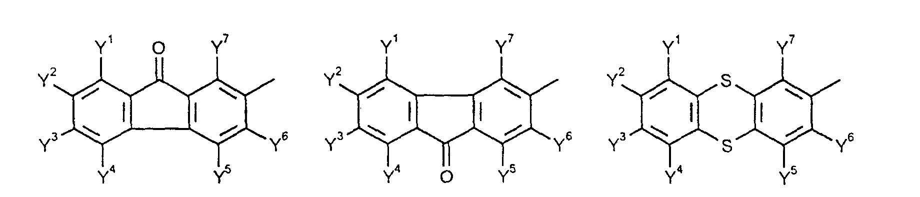 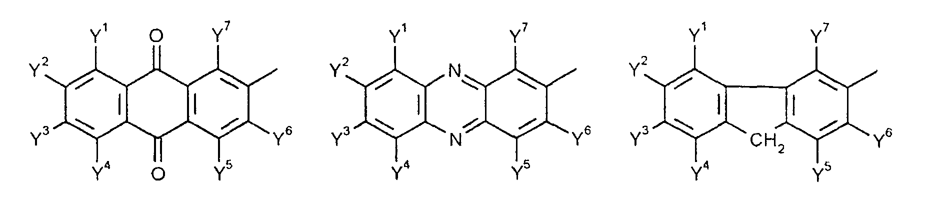 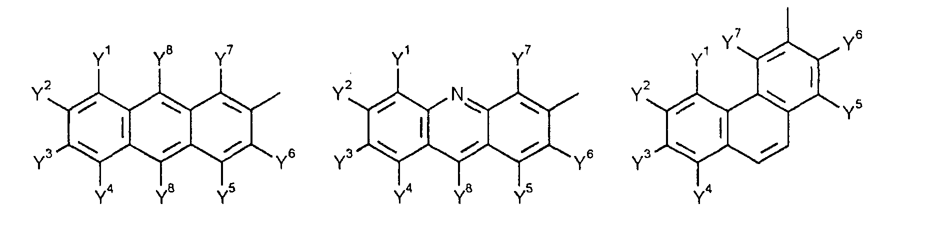 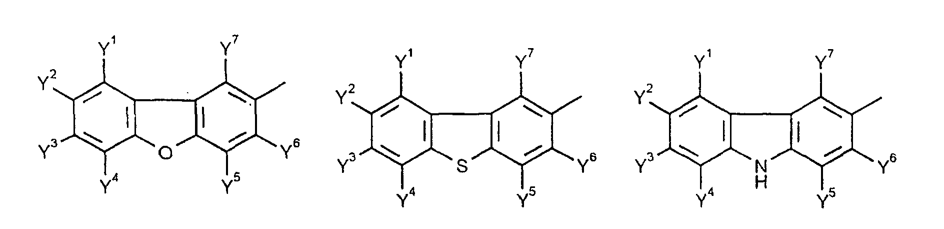 steht, worin
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n-oder i-Propylamino, Dimethylamino, Diethylamino, Cyclopropyl, Cyclobutyl, Cyclopentyl oder Cyclohexyl stehen,
- R²: für Hydroxy, Methoxy oder Ethoxy steht,
- G: für Wasserstoff oder gemeinsam mit R² für ein zweifach gebundenes Sauerstoffatom steht,
- R³: für Wasserstoff oder für Methyl steht,
- R⁴: für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I), in welcher
- A: für eine Einfachbindung oder für Methylen, 1,1-Ethylen, 1,2-Ethylen, 1,1-Propylen, 1,2-Propylen, 1,3-Propylen oder 2,2-Propylen steht,
- Q: für Sauerstoff steht,
- R¹: für eine Gruppierung 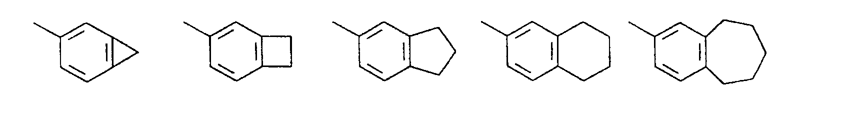 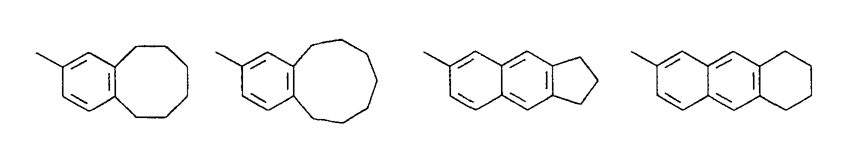 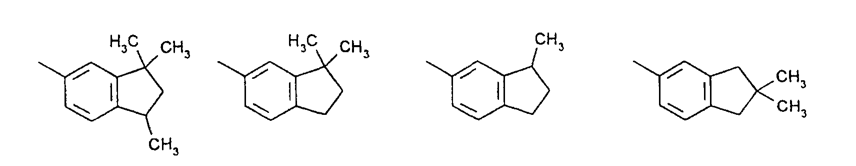 steht, der am Phenyl- bzw. Naphthylteil gegebenenfalls diejenigen Substituenten trägt, die für Y¹ bis Y⁸ genannt sind, oder
- R¹: für eine der nachstehenden Gruppierungen 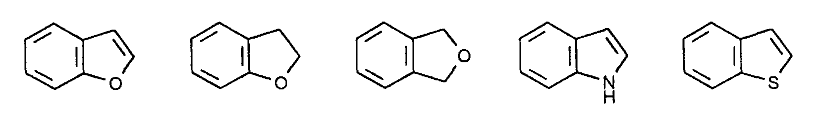 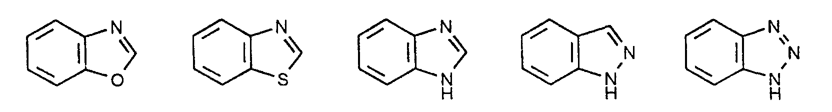 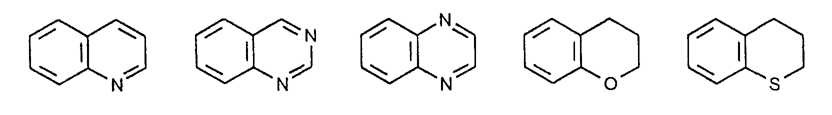 steht, die jeweils über ein Kohlenstoffatom des jeweiligen Benzolringes verbunden sind und die jeweils gegebenenfalls einfach bis dreifach, gleich oder verschieden durch diejenigen Substituenten substituiert sein können, die für Y¹ bis Y⁸ genannt sind, oder
- R¹: für einen Tricyclus 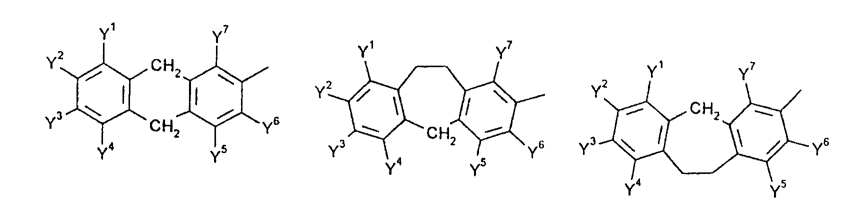 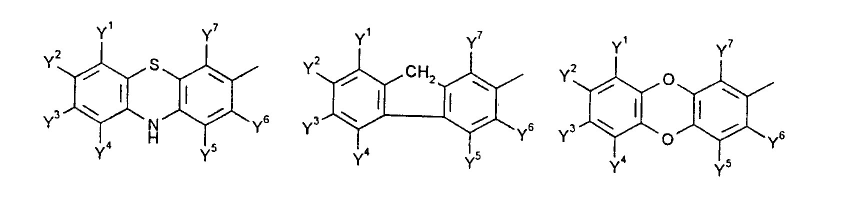 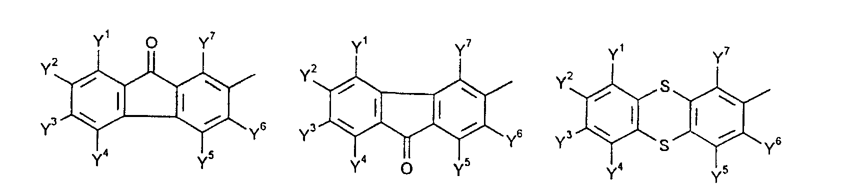 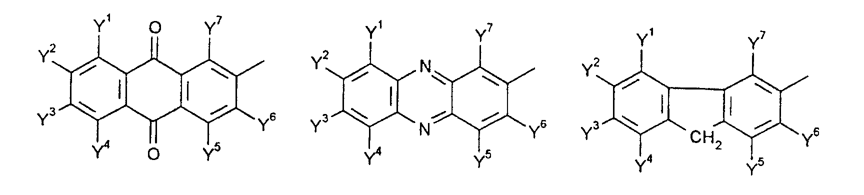 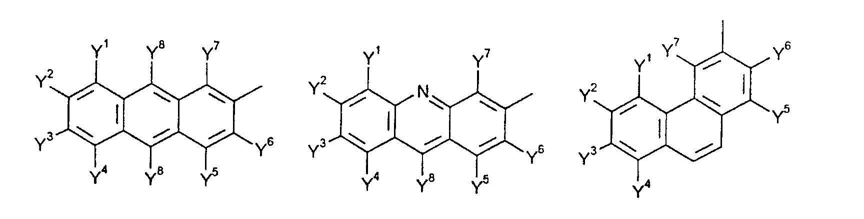 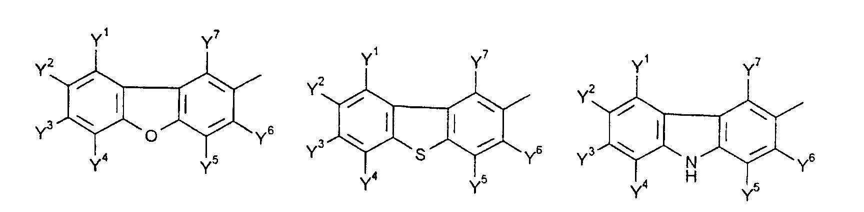 steht, worin
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n-oder i-Propylamino, Dimethylamino, Diethylamino, Cyclopropyl, Cyclobutyl, Cyclopentyl oder Cyclohexyl stehen,
- R²: für Hydroxy, Methoxy oder Ethoxy steht,
- G: für Wasserstoff oder gemeinsam mit R² für ein zweifach gebundenes Sauerstoffatom steht,
- R³: für Wasserstoff oder Methyl steht,
- R⁴: für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

Insbesondere sind auch Verbindungen der Formel (I) bevorzugt, in denen
- R²: für Hydroxy steht.
Insbesondere sind auch Verbindungen der Formel (I) bevorzugt, in denen
- R³: für Wasserstoff steht.

Insbesondere sind auch Verbindungen der Formel (I) bevorzugt, in denen R⁴ für in 3- und 4-Stellung durch Methoxy substituiertes Phenyl steht.

In einer weiterhin besonders bevorzugten Gruppe von Verbindungen steht A für -CH₂-CH₂-.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die in den jeweiligen Kombinationen bzw. bevorzugten Kombinationen von Resten im einzeln für diese Reste angegebenen Restedefinitionen werden unabhängig von der jeweilig angegebenen Kombination, beliebig auch durch Restedefinitionen anderer Vorzugsbereiche ersetzt.

Beispiele für die erfindungsgemäßen Verbindungen sind in der Tabellen 1 aufgeführt:

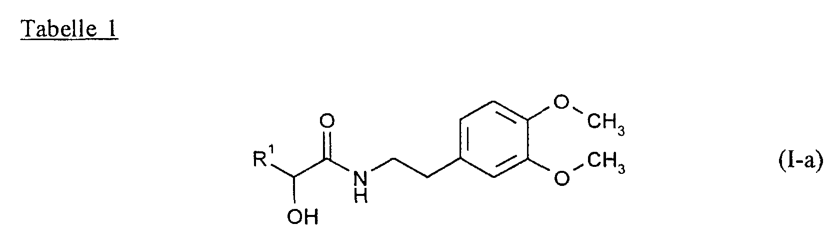

wobei R¹ für die folgenden Substituenten steht:

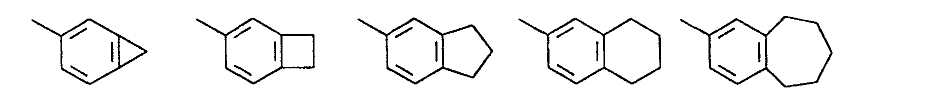

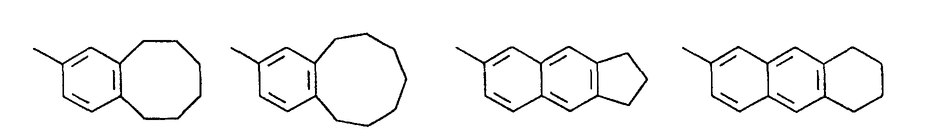

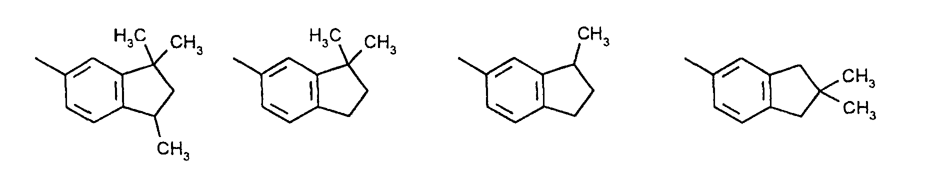

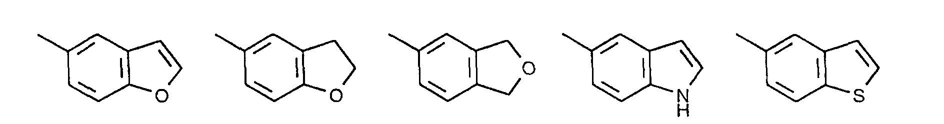

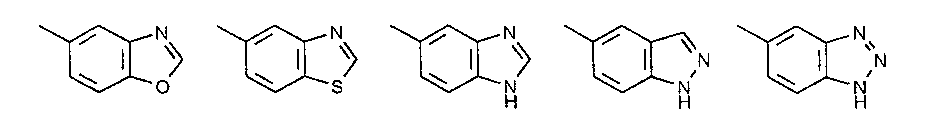

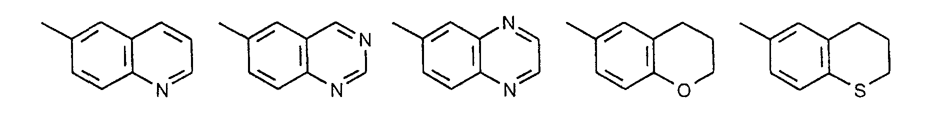

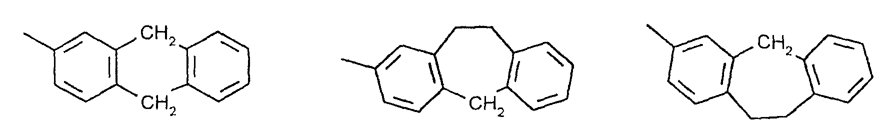

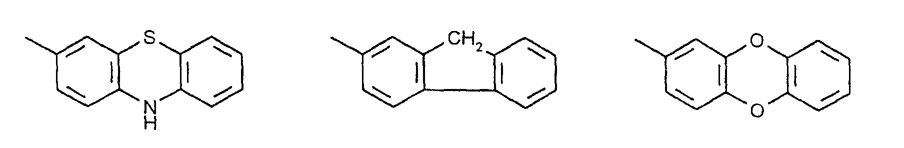

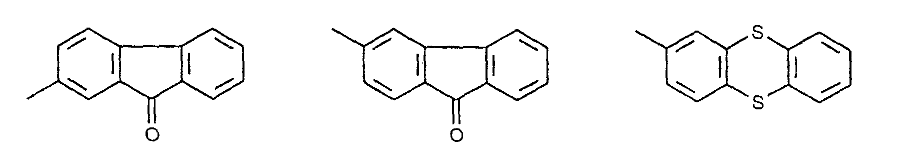

Weiterhin wurde gefunden, daß man die neuen Amide der allgemeinen Formel (I) erhält, wenn man
a) Glyoxylsäureester der Formel (II), 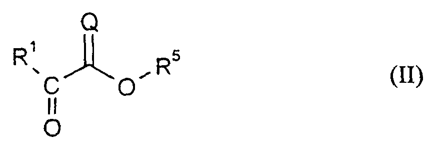 in welcher
   - Q und R¹: die oben angegebenen Bedeutungen haben und
   - R⁵: für Alkyl steht,
   mit einem Amin der Formel (III), 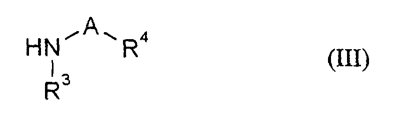 in welcher
   A, R³ und R⁴ die oben angegebenen Bedeutungen haben, gegebenenfalls in Gegenwart eines Verdünnungsmittels und gegebenenfalls in Gegenwart einer Base, umsetzt, oder wenn man
b) Glyoxylsäureamide der Formel (I-b), 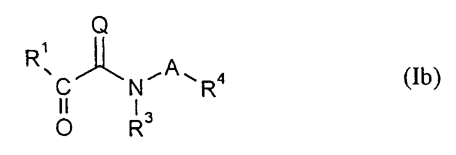 in welcher
   A, Q, R¹ R³ und R⁴ die oben angegebenen Bedeutungen haben,
   mit Wasserstoff oder einem unedlen Metall, gegebenenfalls in Gegenwart eines Katalysators und gegebenenfalls in Gegenwart eines weiteren Reaktionshilfsmittels, umsetzt.

Die zur Durchführung des erfindungsgemäßen Verfahrens als Ausgangsstoffe benötigten Glyoxylsäureester sind durch die Formel (II) allgemein definiert. In dieser Formel (II) haben Q und R¹ vorzugsweise insbesondere diejenige Bedeutungen, die bereits im Zusammenhang mit der Beschreibung der erfindungsgemäßen Verbindungen der Formel (I) als bevorzugt bzw. als insbesondere bevorzugt für Q und R¹ angegeben wurden. R⁵ steht für Alkyl, vorzugsweise für Methyl oder Ethyl.

Die Glyoxylsäureester der Formel (II) sind bekannt und/oder können nach an sich bekannten Verfahren hergestellt werden (vergleiche z. B. Chem.Ber., 76 <1943> 308, 313; Blicke; J.Amer.Chem.Soc., 66 <1944> 1087, 1089; US-P 4150031; DE-A 949521).

Die weiterhin zur Durchführung des erfindungsgemäßen Verfahrens als Ausgangsstoffe benötigten Amine sind durch die Formel (III) allgemein definiert. In dieser Formel (III) haben R³, R⁴ und A vorzugsweise, bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für R³, R⁴ und A angegeben wurden.

Die Amine der Formel (III) sind bekannte organische Synthesechemikalien und/ oder können nach an sich bekannten Verfahren hergestellt werden.

Die zur Durchführung des erfindungsgemäßen Verfahrens b) als Ausgangsstoffe benötigten Glyoxylsäureamide der Formel (I-b) sind erfindungsgemäße Verbindungen und können nach Verfahren a) hergestellt werden.

Als Reduktionsmittel zur Durchführung des erfindungsgemäßen Verfahrens b) kommen alle für derartige Reaktionen übliche Stoffe infrage. Vorzugsweise seien genannt: Wasserstoff, vorzugsweise in Gegenwart eines Katalysators oder unedle Metalle, wie beispielsweise Zink, Zinn, Eisen, Aluminium oder Magnesium.

Als Verdünnungsmittel zur Durchführung des erfindungsgemäßen Verfahrens a) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methylisobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n-oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren a) wird gegebenenfalls in Gegenwart einer geeigneten Base durchgeführt. Als solche kommen alle üblichen anorganischen oder organischen Basen infrage. Hierzu gehören vorzugsweise Erdalkalimetall- oder Alkalimetallalkoholate, wie beispielsweise Natrium-methylat, Natrium-ethylat, Kalium-tert.-butylat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens a) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens a) zur Herstellung der Verbindungen der Formel (I) setzt man pro Mol des Glyoxylsäureesters der Formel (II) im allgemeinen 0,5 bis 15 Mol, vorzugsweise 0,8 bis 2 Mol Amin der Formel (III) ein.

Als Verdünnungsmittel zur Durchführung des erfindungsgemäßen Verfahrens b) kommen alle inerten organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ester wie Essigsäuremethylester oder Essigsäureethylester; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren b) wird gegebenenfalls in Gegenwart eines Katalysators durchgeführt. Als solche kommen alle Katalysatoren infrage, die auch für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Das erfindungsgemäße Verfahren b) wird gegebenenfalls in Gegenwart eines weiteren Reaktionshilfsmittels durchgeführt. Als solche kommen Salze, wie beispielsweise Natriumchlorid, infrage.

Die Reaktionstemperaturen können bei der Durchführung des erfindungsgemäßen Verfahrens b) in einem größeren Bereich variiert werden. Im allgemeinen arbeitet man bei Temperaturen von 0°C bis 150°C, vorzugsweise bei Temperaturen von 20°C bis 100°C.

Die erfindungsgemäßen Verfahren a) und b) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakteria, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankhe!iten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Gemüsebau, wie beispielsweise gegen Phythophthora infestans einsetzen. Ferner lassen sich die erfindungsgemäßen Verbindungen auch zur Steigerung des Ernteertrags von Kulturpflanzen einsetzen.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetate, Iodocarb, Ipconazol, Iprobenfos IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und
Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozcen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofosmethyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)ethyl]amino]carbonyl]propyl}-carbaminsäure-1-isopropylester
1-(2,4-Dichlorphenyl)-2-(1H-1,2,4-triazol-1-yl)ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)methoxy]phenyl]ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichl or-N-[1-(4-chlorphenyl)ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)phenyl]methyl ]-benzamid,
2-(2,3,3-Triiod-2-propenyl)-2H-tetrazol,
2-[(1-Methylethyl)sulfonyl]-5-(trichlormethyl)-1,3,4-thiadiazol,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)oxy]methyl]-benzamid,
3 -(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol, -Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis(2-propinyloxy)-phenyl]-N'-methoxy-methanimidamid,
N-Formyl-N-hydroxy-DL-alanin, -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methylethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihren handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw.. Es ist ferner möglich die Wirkstoffe nach dem Ultra-Low-Volume-Verfahren auszubringen oder die Wirkstoffzubereitung oder der Wirkstoff selbst in den Boden zu injizieren. Es kann wird auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1

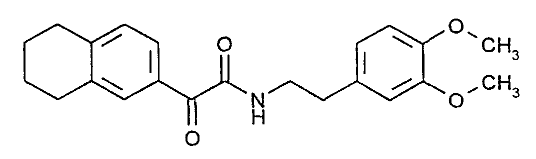

Zu einer Lösung von 11,6 g (0,05 Mol) Oxo-(5,6,7,8-tetrahydro-naphthalin-2-yl)-essigsäuremethylester und 9,06 g (0,05 Mol) 2-(3,4-Dimethoxyphenyl)-ethylamin in 100 ml Methanol gibt man 18 g (0,1 Mol) 30%ige, methanolische Natriummethanolatlösung und rührt 2 Stunden bei 65°C. Die Reaktionslösung wird bei vermindertem Druck eingeengt und der Rückstand in Dichlormethan aufgenommen. Diese Lösung wird zuerst mit Wasser, dann mit 1 N Salzsäure und wiederum mit Wasser gewaschen, über Natriumsulfat getrocknet und bei vermindertem Druck eingeengt. Der Rückstand wird mit Petrolether/Essigester (2:1) an Kieselgel chromatografiert. Man erhält 6,4 g (35 % der Theorie) N-[2-(3,4-Dimethoxyphenyl)-ethyl]-2-oxo-2-(5,6,7,8-tetrahydronaphthalin-2-yl)-acetamid.
logP: 3,24

### Beispiel 2

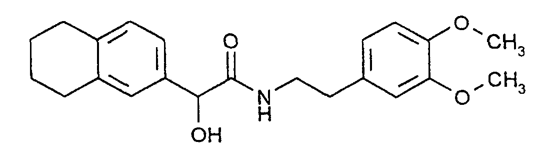

Zu einer Lösung von 2,5 g (6,8 mMol) N-[2-(3,4-Dimethoxyphenyl)-ethyl]-2-oxo-2-(5,6,7,8-tetrahydro-naphthalin-2-yl)-acetamid in 50 ml Methanol gibt man nacheinander 50 ml Wasser, 2,5 g Zinkstaub und 2,5 g Natriumchlorid und kocht 30 Stunden unter Rückfluß. Die Reaktionslösung wird bei vermindertem Druck eingeengt und der Rückstand in Dichlormethan aufgenommen. Diese Lösung wird zuerst mit Wasser, dann mit 1 N Salzsäure und wiederum mit Wasser gewaschen, über Natriumsulfat getrocknet und bei vermindertem Druck eingeengt. Man erhält 1,8 g (72 % der Theorie) N-[2-(3,4-Dimethoxyphenyl)-ethyl]-2-hydroxy-2-(5,6,7,8-tetrahydronaphthalin-2-yl)-acetamid.
logP: 2,43

Analog Beispiel 1 sowie entsprechend der allgemeinen Beschreibung des erfindungsgemäßen Herstellungsverfahrens, können beispielsweise auch die in der nachstehenden Tabelle 2 aufgeführten Verbindungen hergestellt werden:

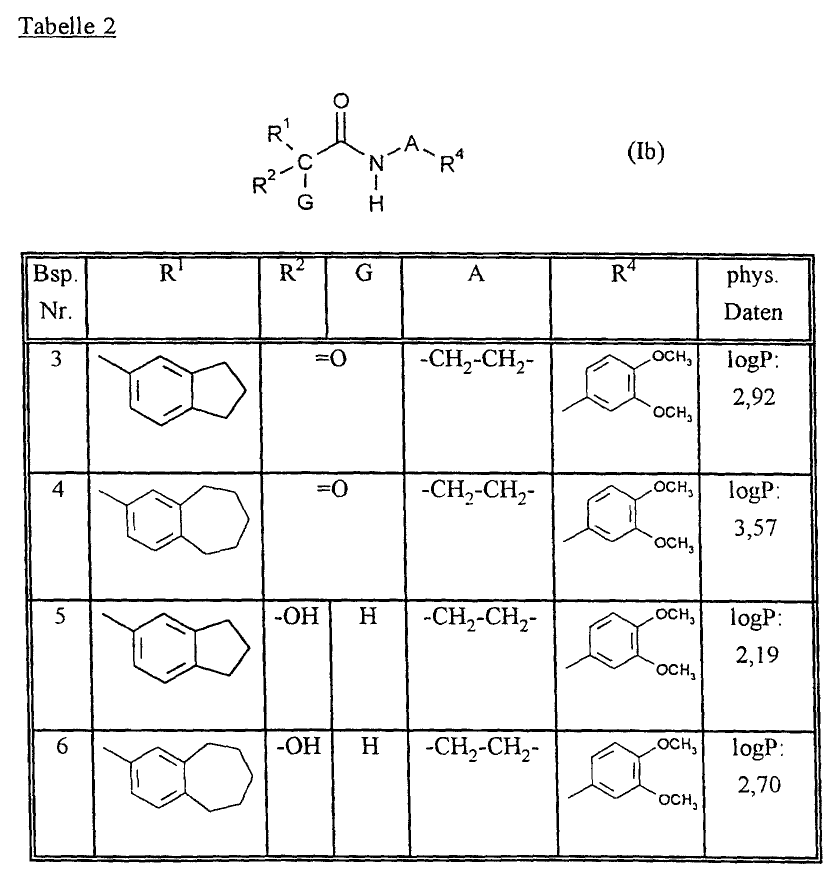

### Anwendungsbeispiele:

### Beispiel

| Phytophthora-Test (Tomate) / protektiv | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert.

Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100 % bedeutet, daß kein Befall beobachtet wird.

Bei diesem Test zeigen z.B. folgenden Verbindungen (1) und (3) der Herstellungsbeispiele bei einer beispielhaften Wirkstoffaufwandmenge von 50 g/ha einen Wirkungsgrad von 90 -95 % der unbehandelten Kontrolle.

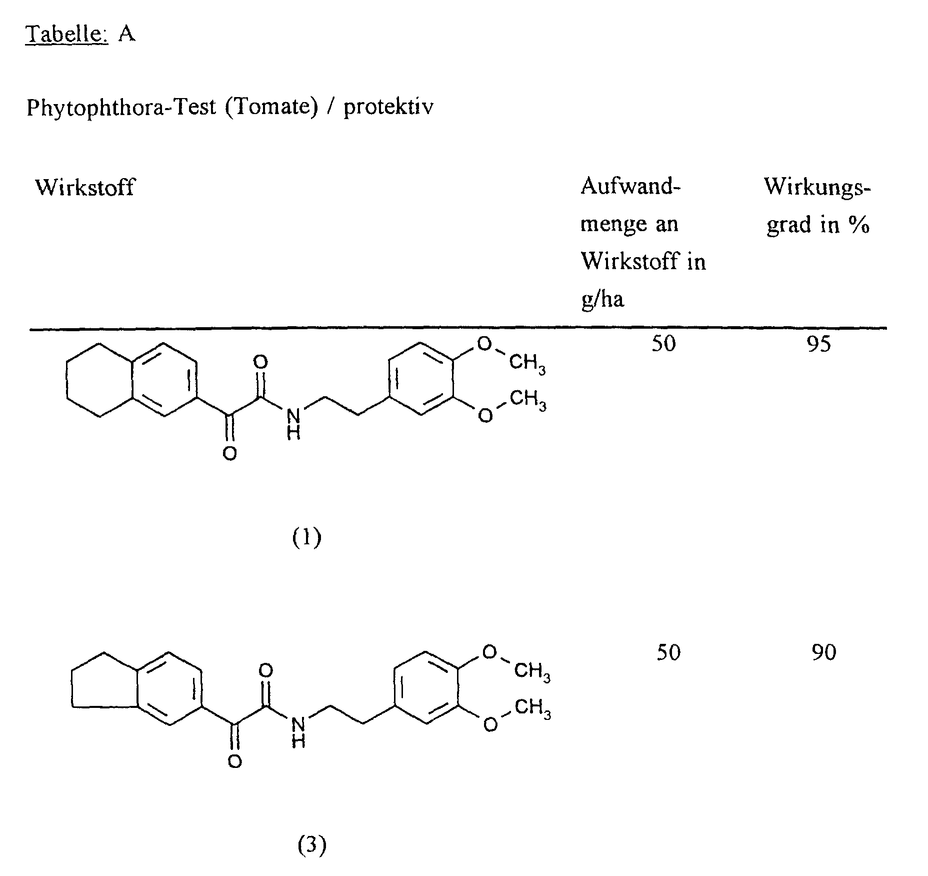

## Patentansprüche

1. Verbindungen der Formel (I), 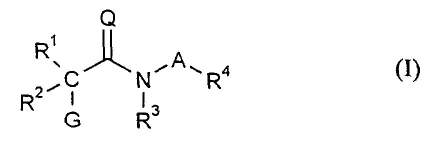 in welcher
A für eine Einfachbindung oder gegebenenfalls substituiertes Alkylen steht,
Q für Sauerstoff oder Schwefel steht,
R¹ für Aryl steht, an das ein Cycloalkylring ankondensiert ist, wobei sowohl der Arylteil als auch der Cycloalkylteil gegebenenfalls weitere Substituenten trägt, oder
R¹ für gegebenenfalls substituiertes, am Benzolring verknüpftes, Benzoheterocyclyl mit einem, zwei oder drei Heteroatomen steht, oder
R¹ für einen Tricyclus 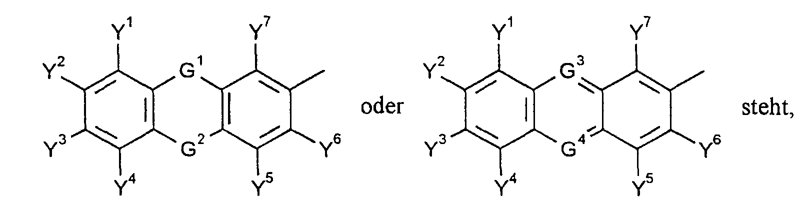 worin
G¹ und G² unabhängig voneinander für eine Einfachbindung, Alkandiyl, Alkendiyl, Sauerstoff, Schwefel, -NH-, -N(Alkyl)- oder Carbonyl stehen,
G³ und G⁴ unabhängig voneinander für Stickstoff oder eine Gruppierung 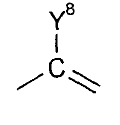 stehen und
Y¹, Y², Y³, Y⁴, Y⁵, Y⁶, Y⁷ und Y⁸ unabhängig voneinander für Wasserstoff, Halogen, Cyano, Nitro, jeweils gegebenenfalls substituiertes Alkyl, Alkoxy, Alkylamino, Dialkylamino, Alkylthio, Alkylsulfinyl, Alkylsulfonyl oder Cycloalkyl stehen,
R² für Hydroxy oder Alkoxy steht,
G für Wasserstoff oder gemeinsam mit R² für ein zweifach gebundenes Sauerstoffatom steht,
R³ für Wasserstoff oder jeweils gegebenenfalls substituiertes Alkyl, Alkenyl, Alkinyl oder Cycloalkyl steht,
R⁴ für jeweils gegebenenfalls substituiertes Cycloalkyl, Cycloalkenyl, Aryl oder Heterocyclyl steht.

2. Verbindungen der Formel (I) gemäß Anspruch 1, in welcher
A für eine Einfachbindung oder für gegebenenfalls einfach oder mehrfach, gleich oder verschieden substituiertes Alkylen mit 1 bis 6 Kohlenstoffatomen steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl,
Q für Sauerstoff oder Schwefel steht,
R¹ für Phenyl oder Naphthyl steht, an das ein Cycloalkylring mit 3 bis 10 Ringgliedern ankondensiert ist, wobei der Cycloalkylteil gegebenenfalls durch 1 bis 4 Alkylketten mit jeweils 1 - 4 Kohlenstoffatomen substituiert ist und der Phenyl- bzw. Naphthylteil gegebenenfalls diejenigen Substituenten trägt, die für Y¹ bis Y⁸ genannt sind, oder
R¹ für gegebenenfalls einfach oder mehrfach, gleich oder verschieden substituiertes, am Benzolring verknüpftes, Benzoheterocyclyl mit 3 bis 12 Ringgliedern im Heterocyclylteil und einem, zwei oder drei Heteroatomen steht, wobei die möglichen Substituenten diejenigen sind, die für Y¹ bis Y⁸ genannt sind, oder
R¹ für einen Tricyclus 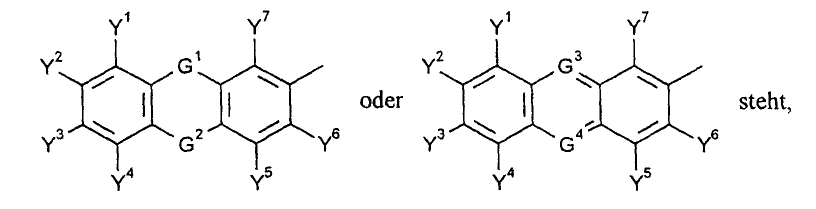 worin
G¹ und G² unabhängig voneinander für eine Einfachbindung, Alkandiyl, Alkendiyl, Sauerstoff, Schwefel, -NH-, -N(Alkyl)- oder Carbonyl stehen,
G³ und G⁴ unabhängig voneinander für Stickstoff oder eine Gruppierung 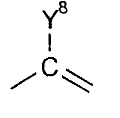 stehen und
Y¹, Y², Y³, Y⁴, Y⁵, Y⁶, Y⁷ und Y⁸ unabhängig voneinander für Wasserstoff, Halogen, Cyano, Nitro;
Alkyl, Alkoxy, Alkylamino, Dialkylamino, Alkylthio, Alkylsulfinyl oder Alkylsulfonyl mit jeweils 1 bis 8 Kohlenstoffatomen in den einzelnen Alkylteilen;
Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen oder
Cycloalkyl mit 3 bis 6 Kohlenstoffatomen stehen,
R² für Hydroxy oder Alkoxy mit 1 bis 4 Kohlenstoffatomen steht,
G für Wasserstoff oder gemeinsam mit R² für ein zweifach gebundenes Sauerstoffatom steht,
R³ für Wasserstoff oder Alkyl mit 1 bis 4 Kohlenstoffatomen steht,
R⁴ für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

3. Verbindungen der Formel (I) gemäß Anspruch 1, in welcher
A für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3-oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für eine Gruppierung 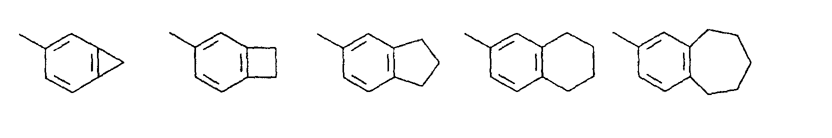 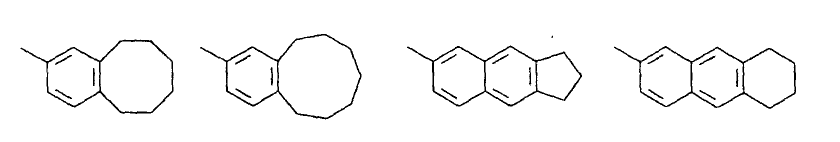 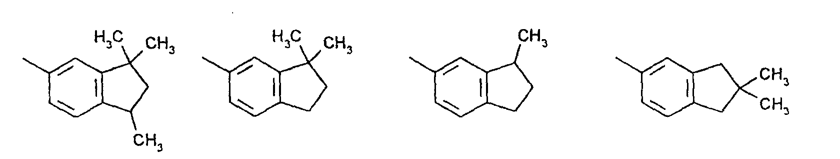 steht, der am Phenyl- bzw. Naphthylteil gegebenenfalls diejenigen Substituenten trägt, die für Y¹ bis Y⁸ genannt sind, oder
R¹ für eine der nachstehenden Gruppierungen 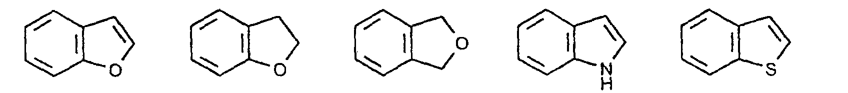 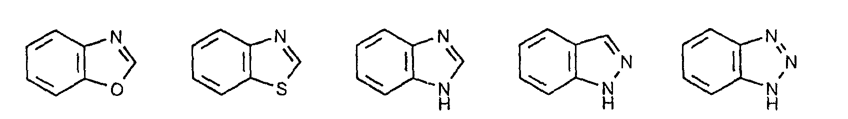 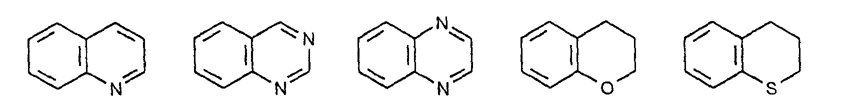 steht, die jeweils über ein Kohlenstoffatom des jeweiligen Benzolringes verbunden sind und die jeweils gegebenenfalls einfach bis dreifach, gleich oder verschieden durch diejenigen Substituenten substituiert sein können, die für Y¹ bis Y⁸ genannt sind, oder
R¹ für einen Tricyclus 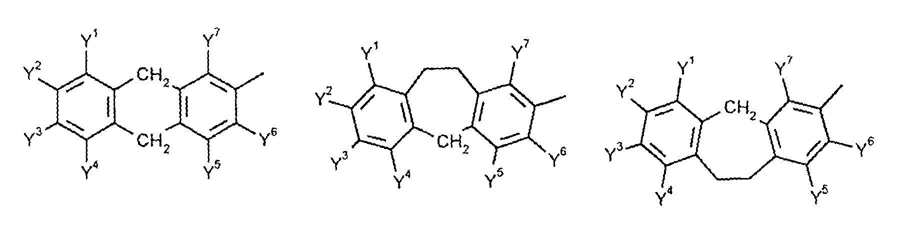 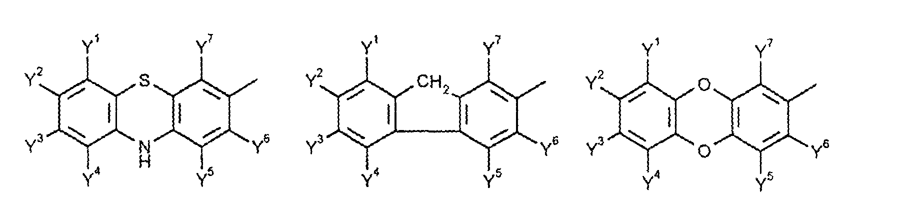 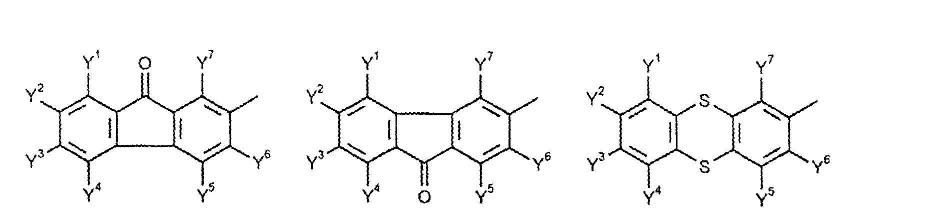 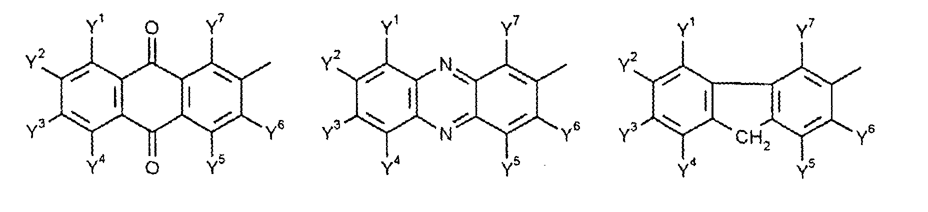 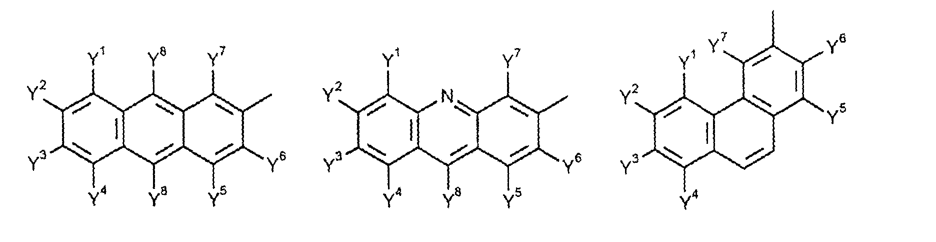 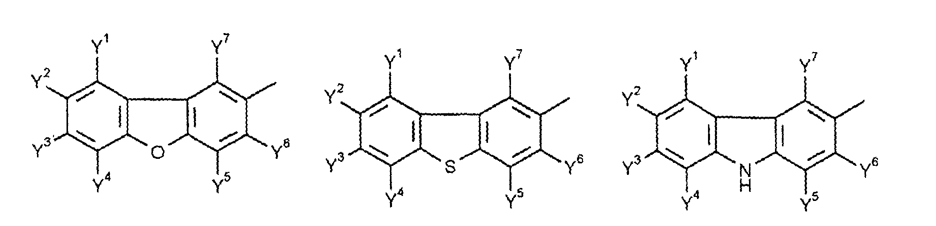 steht, worin
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Cyclopropyl, Cyclobutyl, Cyclopentyl oder Cyclohexyl stehen,
R² für Hydroxy, Methoxy oder Ethoxy steht,
G für Wasserstoff oder gemeinsam mit R² für ein zweifach gebundenes Sauerstoffatom steht,
R³ für Wasserstoff oder für Methyl steht,
R⁴ für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

4. Verbindungen der Formel (I) gemäß Anspruch 1, in welcher
A für eine Einfachbindung oder für Methylen, 1,1-Ethylen, 1,2-Ethylen, 1,1-Propylen, 1,2-Propylen, 1,3-Propylen oder 2,2-Propylen steht,
Q für Sauerstoff steht,
R¹ für eine Gruppierung 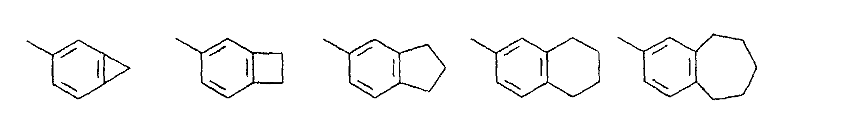 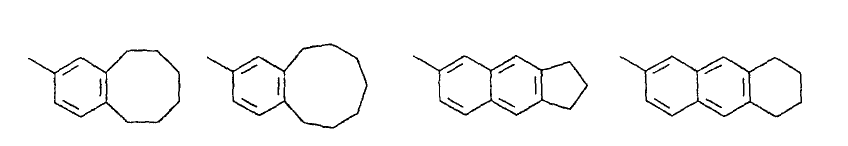 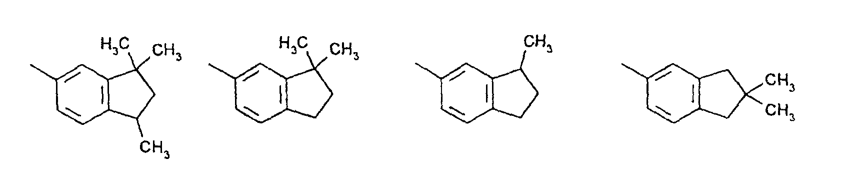 steht, der am Phenyl- bzw. Naphthylteil gegebenenfalls diejenigen Substituenten trägt, die für Y¹ bis Y⁸ genannt sind, oder
R¹ für eine der nachstehenden Gruppierungen 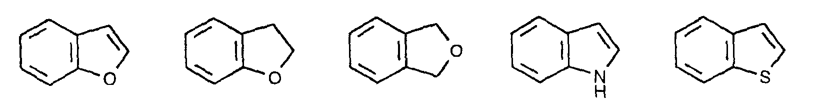 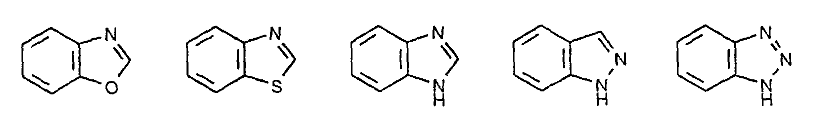 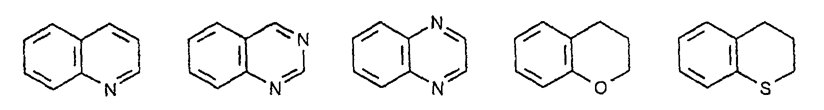 steht, die jeweils über ein Kohlenstoffatom des jeweiligen Benzolringes verbunden sind und die jeweils gegebenenfalls einfach bis dreifach, gleich oder verschieden durch diejenigen Substituenten substituiert sein können, die für Y¹ bis Y⁸ genannt sind, oder
R¹ für einen Tricyclus 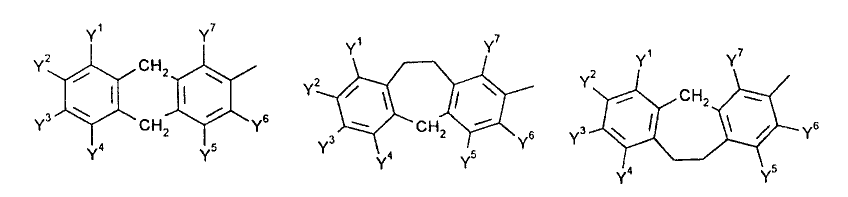 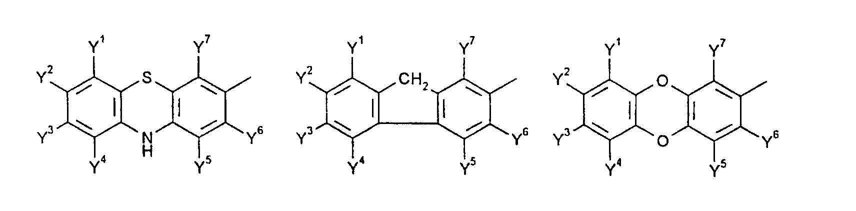 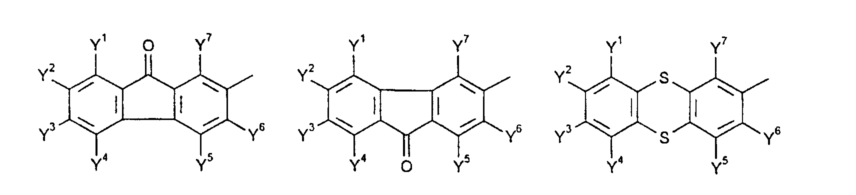 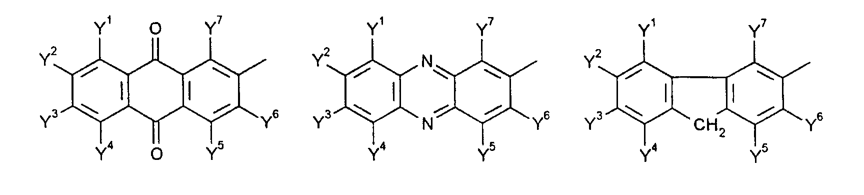 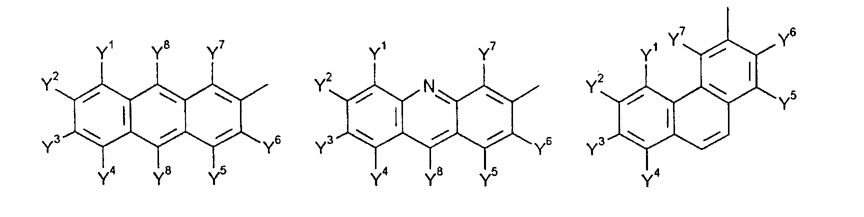 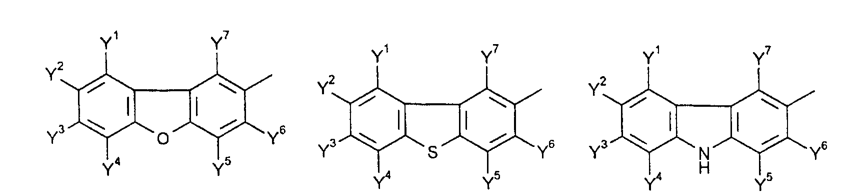 steht, worin
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Cyclopropyl, Cyclobutyl, Cyclopentyl oder Cyclohexyl stehen,
R² für Hydroxy, Methoxy oder Ethoxy steht,
G für Wasserstoff oder gemeinsam mit R² für ein zweifach gebundenes Sauerstoffatom steht,
R³ für Wasserstoff oder Methyl steht,
R⁴ für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

5. Verbindungen der Formel (I) gemäß Anspruch 1, in denen
R² für Hydroxy steht.

6. Schädlingsbekämpfungsmittel, **gekennzeichnet durch** einen Gehalt an mindestens einer Verbindung der Formel (I) nach Anspruch 1.

7. Verfahren zur Bekämpfung von Schädlingen, **dadurch gekennzeichnet, daß** man Verbindungen der Formel (I) nach Anspruch 1 auf Schädlinge und/ oder ihren Lebensraum einwirken läßt.

8. Verfahren zur Herstellung von Verbindungen der Formel (I), **dadurch gekennzeichnet, daß** man
a) Glyoxylsäureester der Formel (II), 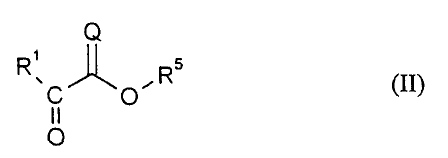 in welcher
Q und R¹ die oben angegebenen Bedeutungen haben und
R⁵ für Alkyl steht,
mit einem Amin der Formel (III), 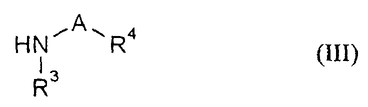 in welcher
A, R³ und R⁴ die oben angegebenen Bedeutungen haben,
gegebenenfalls in Gegenwart eines Verdünnungsmittels und gegebenenfalls in Gegenwart einer Base, umsetzt, oder
b) Glyoxylsäureamide der Formel (I-b), 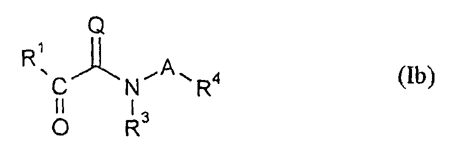 in welcher
A, Q, R¹ R³ und R⁴ die oben angegebenen Bedeutungen haben,
mit Wasserstoff oder einem unedlen Metall, gegebenenfalls in Gegenwart eines Katalysators und gegebenenfalls in Gegenwart eines weiteren Reaktionshilfsmittels, umsetzt.

9. Verwendung von Verbindungen der Formel (I) nach den Ansprüchen 1 bis 8 zur Bekämpfung von Schädlingen.

10. Verfahren zur Herstellung von Schädlingsbekämpfungsmitteln, **dadurch gekennzeichnet, daß** man Verbindungen der Formel (I) nach den Ansprüchen 1 bis 8 mit Streckmitteln und/oder oberflächenaktiven Mitteln vermischt.

## Claims

1. Compounds of the formula (I), 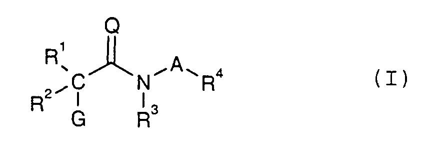 in which
A represents a single bond or optionally substituted alkylene,
Q represents oxygen or sulphur,
R¹ represents aryl having a fused-on cycloalkyl ring, where both the aryl moiety and the cycloalkyl moiety optionally carry further substitutents, or
R¹ represents optionally substituted benzoheterocyclyl which is attached at the benzene ring and has one, two or three heteroatoms, or
R¹ represents a tricycle 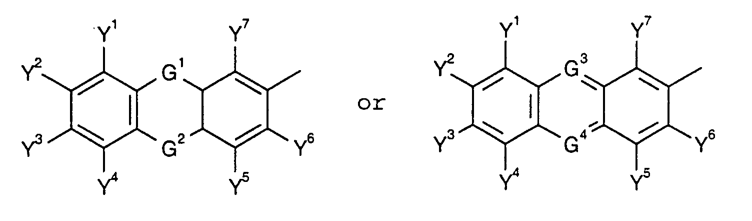 in which
G¹ and G² independently of one another represent a single bond, alkanediyl, alkenediyl, oxygen, sulphur, -NH-,-N(alkyl)- or carbonyl,
G³ and G⁴ independently of one another represent nitrogen or a grouping 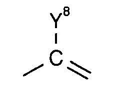 and
Y¹, Y², Y³, Y⁴, Y⁵, Y⁶, Y⁷ and Y⁸ independently of one another represent hydrogen, halogen, cyano, nitro, in each case optionally substituted alkyl, alkoxy, alkylamino, dialkylamino, alkylthio, alkylsulphinyl, alkylsulphonyl or cycloalkyl,
R² represents hydroxyl or alkoxy,
G represents hydrogen or together with R² represents a doubly attached oxygen atom,
R³ represents hydrogen or in each case optionally substituted alkyl, alkenyl, alkinyl or cycloalkyl,
R⁴ represents in each case optionally substituted cycloalkyl, cycloalkenyl, aryl or heterocyclyl.

2. Compounds of the formula (I) according to Claim 1 in which
A represents a single bond or represents alkylene which is optionally mono- or polysubstituted by identical or different substituents and has 1 to 6 carbon atoms, where the possible substituents are preferably selected from the list below:
halogen, cyano, nitro, amino, hydroxyl, formyl, carboxyl, carbamoyl, thiocarbamoyl;
in each case straight-chain or branched alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
cycloalkyl having 3 to 6 carbon atoms;
and aryl or heterocyclyl, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and in each case doubly attached alkylene or dioxyalkylene having in each case 1 to 6 carbon atoms and being optionally mono- or polysubstituted by identical or different substituents selected from -the group consisting of halogen and straight-chain or branched alkyl having 1 to 4 carbon atoms and straight-chain or branched halogenoalkyl having 1 to 4 carbon atoms and 1 to 9 identical or different halogen atoms,
Q represents oxygen or sulphur,
R¹ represents phenyl or naphthyl having a fused-on cycloalkyl ring having 3 to 10 ring members, where the cycloalkyl moiety is optionally substituted by 1 to 4 alkyl chains having in each case 1 - 4 carbon atoms and the phenyl or naphthyl moiety optionally carries those substituents mentioned for Y¹ to Y⁸, or
R¹ represents benzoheterocyclyl which is attached at the benzene ring and has 3 to 12 ring members in the heterocyclyl moiety and one, two or three heteroatoms and is optionally mono- or polysubstituted by identical or different substituents, possible substituents being those which are mentioned for Y¹ to Y⁸, or
R¹ represents a tricycle 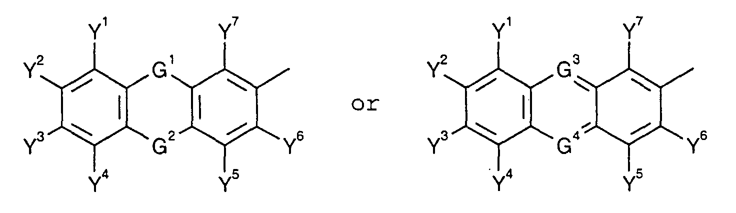 in which
G¹ and G² independently of one another represent a single bond, alkanediyl, alkenediyl, oxygen, sulphur, -NH-,-N(alkyl)- or carbonyl,
G³ and G⁴ independently of one another represent nitrogen or a grouping 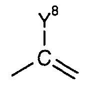 and
Y¹, Y², Y³, Y⁴, Y⁵, Y⁶, Y⁷ and Y⁸ independently of one another represent hydrogen, halogen, cyano, nitro;
alkyl, alkoxy, alkylamino, dialkylamino, alkylthio, alkylsulphinyl or alkylsulphonyl having in each case 1 to 8 carbon atoms in the individual alkyl moieties;
halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms or
cycloalkyl having 3 to 6 carbon atoms,
R² represents hydroxyl or alkoxy having 1 to 4 carbon atoms,
G represents hydrogen or together with R² represents a doubly attached oxygen atom,
R³ represents hydrogen or alkyl having 1 to 4 carbon atoms,
R⁴ represents aryl, cycloalkyl or cycloalkenyl having 3 to 8 carbon atoms, or heterocyclyl having 3 to 12 ring members, each of which is optionally mono- or polysubstituted by identical or different substituents, where the possible substituents are preferably selected from the list below:
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
in each case doubly attached alkylene or dioxyalkylene having in each case 1 to 6 carbon atoms and being in each case optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
and aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio,
each of which is optionally mono- or polysubstituted by identical or different substituents selected from the group consisting of
halogen, cyano and straight-chain or branched alkyl having 1 to 4 carbon atoms,
and straight-chain or branched halogenoalkyl having 1 to 4 carbon atoms and 1 to 9 identical or different halogen atoms,
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms,
and in each case doubly attached alkylene or dioxyalkylene having in each case 1 to 6 carbon atoms and being optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms.

3. Compounds of the formula (I) according to Claim 1 in which
A represents a single bond or represents methylene, 1,1-ethylene, 1,2-ethylene, 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents selected from the group consisting of fluorine, chlorine, cyano and methoxy,
Q represents oxygen or sulphur,
R¹ represents a grouping 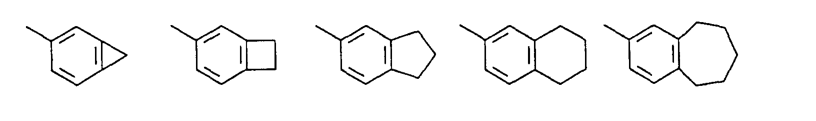 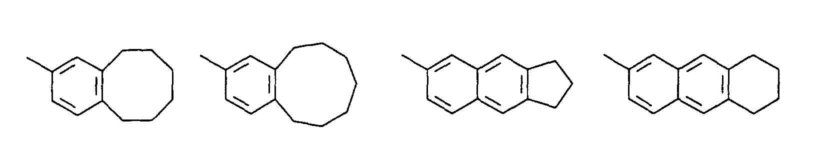 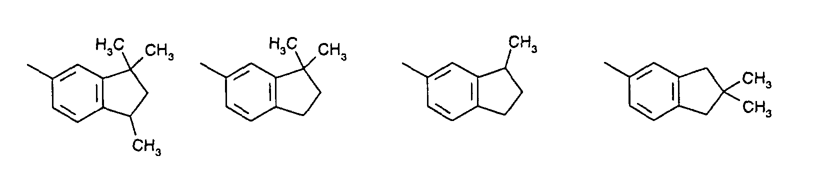 which, at the phenyl or naphthyl moiety, optionally carries those substituents mentioned for Y¹ to Y⁸, or
R¹ represents one of the groupings below 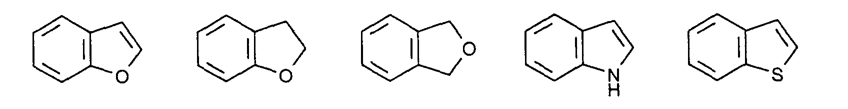 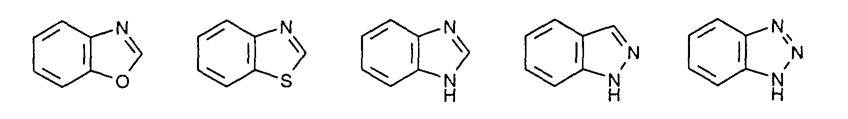 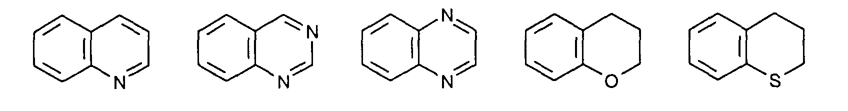 each of which is attached via a carbon atom of the benzene ring in question and each of which may optionally be mono- to trisubstituted by identical or different substituents mentioned for Y¹ to Y⁸, or
R¹ represents a tricycle 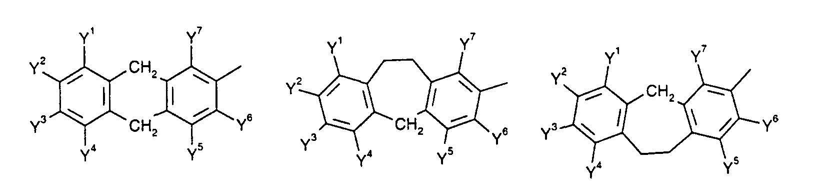 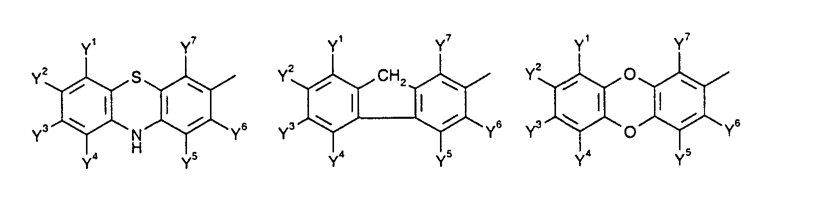 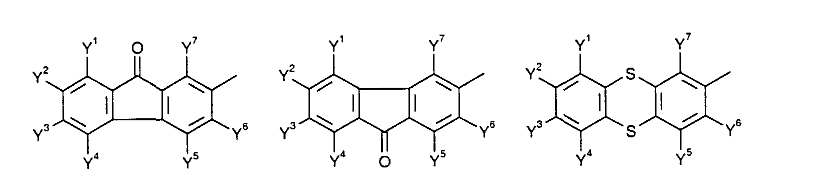 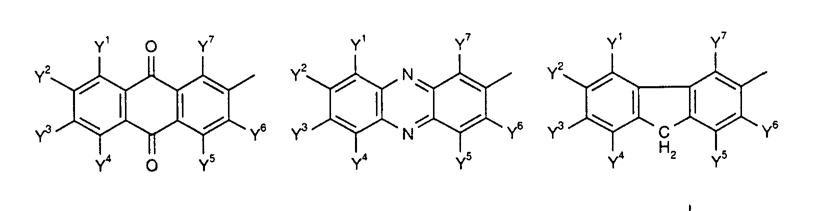 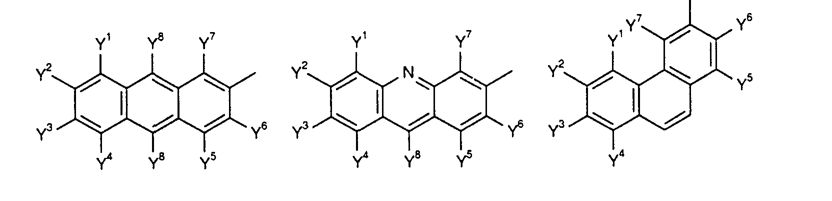 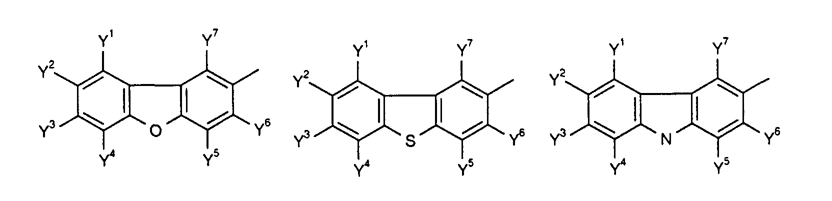 in which
Y¹, Y², Y³, Y⁴, Y⁵, Y⁶, Y⁷ and Y⁸ independently of one another represent hydrogen, fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethyl-sulphonyl, methylamino, ethylamino, n- or i-propylamino, dimethylamino, diethylamino, cyclopropyl, cyclobutyl, cyclopentyl or cyclohexyl,
R² represents hydroxyl, methoxy or ethoxy,
G represents hydrogen or together with R² represents a doubly attached oxygen atom,
R³ represents hydrogen or represents methyl,
R⁴ represents in each case optionally mono- to hexasubstituted cyclopropyl, cyclobutyl, cyclopentyl or cyclohexyl, where the preferred substituents are those mentioned below;
represents in each case optionally mono- to trisubstituted phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, where the possible substituents are preferably selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethyl-sulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl, in each case doubly attached trimethylene (propane-1,3-diyl), tetramethylene (butane-1,4-diyl), methylenedioxy or ethylenedioxy, each of which is optionally mono- or polysubstituted by identical or different substituents selected from the group consisting of fluorine, chlorine, methyl, trifluoromethyl; ethyl, n- and i-propyl,
cyclopropyl, cyclobutyl, cyclopentyl or cyclohexyl.

4. Compounds of the formula (I) according to Claim 1 in which
A represents a single bond or represents methylene, 1,1-ethylene, 1,2-ethylene, 1,1-propylene, 1,2-propylene, 1,3-propylene or 2,2-propylene,
Q represents oxygen,
R¹ represents a grouping 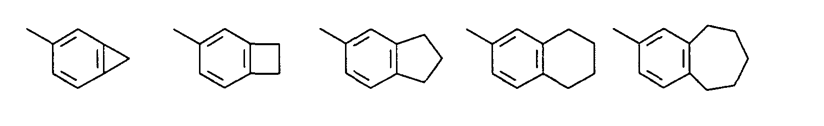 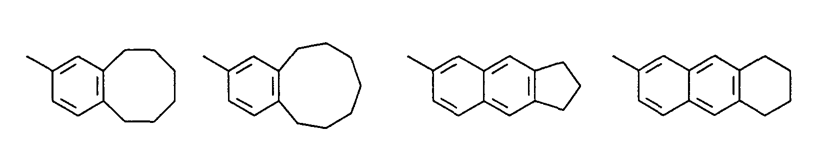 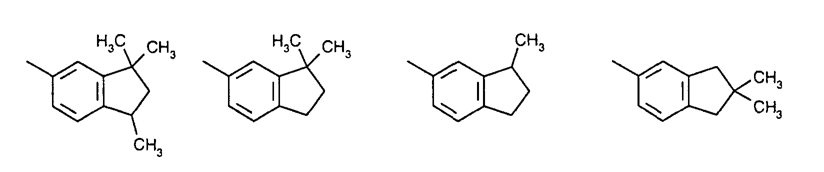 which, at the phenyl or naphthyl moiety, optionally carries those substituents mentioned for Y¹ to Y⁸, or
R¹ represents one of the groupings below 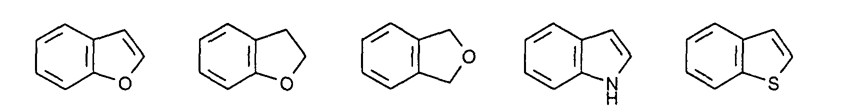 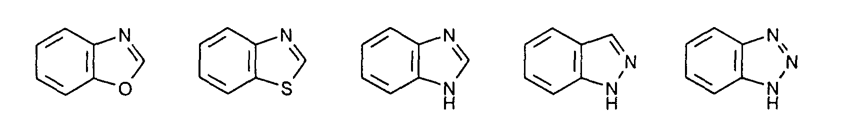 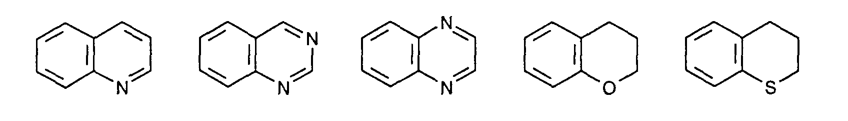 each of which is attached via a carbon atom of the benzene ring in question and each of which may optionally be mono- to trisubstituted by identical or different substituents mentioned for Y¹ to Y⁸, or
R¹ represents a tricycle 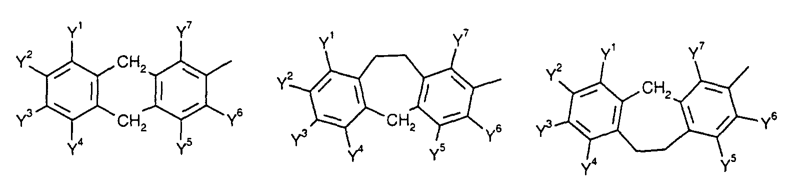 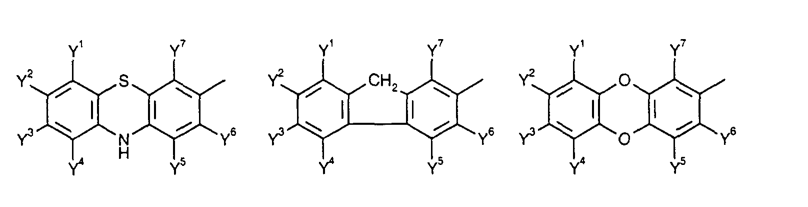 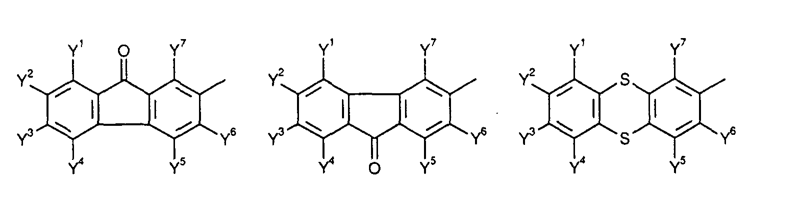 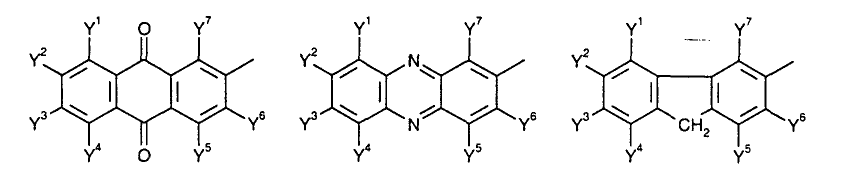 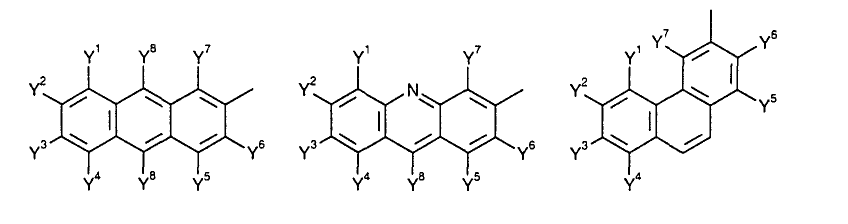 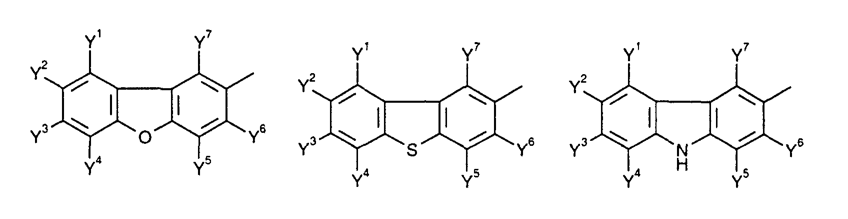 in which
Y¹, Y², Y³, Y⁴, Y⁵, Y⁶, Y⁷ and Y⁸ independently of one another represent hydrogen, fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoro-methoxy, difluorochloromethoxy, trifluoroethoxy, difluoromethylthio, difluorochloromethylthio, trifluoromethylthio, trifluoromethylsulphinyl or trifluoromethylsulphonyl, methyl-amino, ethylamino, n- or i-propylamino, dimethylamino, diethylamino, cyclopropyl, cyclobutyl, cyclopentyl or cyclohexyl,
R² represents hydroxyl, methoxy or ethoxy,
G represents hydrogen or together with R² represents a doubly attached oxygen atom,
R³ represents hydrogen or methyl,
R⁴ represents cyclohexyl or optionally mono- to trisubstituted phenyl, thienyl, furyl, benzofuryl, benzothienyl, pyridyl, pyrimidinyl, naphthyl or quinolyl, where the possible substituents are preferably selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoro-ethoxy, difluoromethylthio, difluorochloromethylthio, trifluoro-methylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydrox-iminomethyl, hydroximinoethyl, methoximinomethyl, ethoximinomethyl, methoximinoethyl or ethoximinoethyl,
in each case doubly attached trimethylene (propane-1,3-diyl), tetramethylene (butane-1,4-diyl), methylenedioxy or ethylenedioxy, each of which is optionally mono- or polysubstituted by identical or different substituents selected from the group consisting of fluorine, chlorine, methyl, trifluoromethyl, ethyl, n- and i-propyl.

5. Compounds of the formula (I) according to Claim 1 in which
R² represents hydroxyl.

6. Pesticides, **characterized in that** they contain at least one compound of the formula (I) according to Claim 1.

7. Method for controlling pests, **characterized in that** compounds of the formula (I) according to Claim 1 are allowed to act on pests and/or their habitat.

8. Process for preparing compounds of the formula (I), **characterized in that**
a) glyoxylic esters of the formula (II), 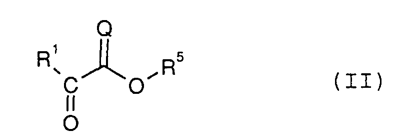 in which
Q and R¹ are as defined above and
R⁵ represents alkyl
are reacted with an amine of the formula (III), 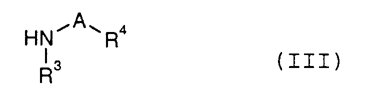 in which
A, R³ and R⁴ are as defined above,
if appropriate in the presence of a diluent and if appropriate in the presence of a base, or
b) glyoxylic acid amides of the formula (I-b), 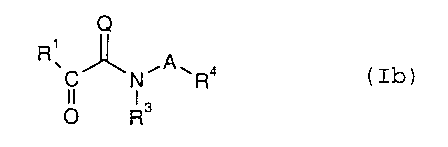 in which
A, Q, R¹, R³ and R⁴ are as defined above,
are reacted with hydrogen or a base metal, if appropriate in the presence of a catalyst and if appropriate in the presence of a further reaction auxiliary.

9. The use of compounds of the formula (I) according to Claims 1 to 8 for controlling pests.

10. Process for preparing pesticides, **characterized in that** compounds of the formula (I) according to Claims 1 to 8 are mixed with extenders and/or surfactants.

## Revendications

1. Composés répondant à la formule (I) 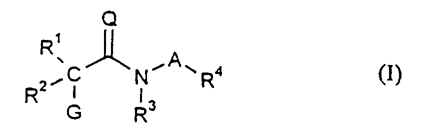 dans laquelle
A représente une liaison simple ou un groupe alkylène le cas échéant substitué
Q représente un atome d'oxygène ou un atome de soufre,
R¹ représente un groupe aryle auquel est condensé un noyau cycloalkyle, aussi bien la fraction aryle que la fraction cycloalkyle portant le cas échéant d'autres substituants, ou
R¹ représente un groupe benzohétérocyclyle le cas échéant substitué, lié au noyau benzénique, contenant un, deux ou trois hétéroatomes, ou
R¹ représente un groupement tricyclique 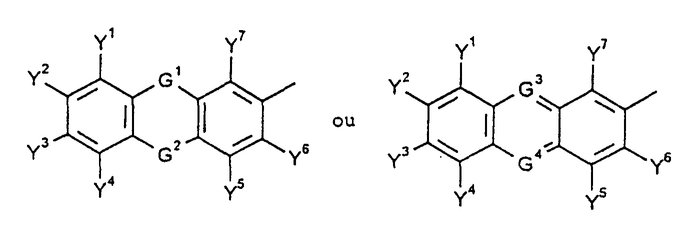 dans lequel
G¹ et G² représentent, indépendamment l'un de l'autre, une liaison simple, un groupe alcanediyle, un groupe alcènediyle, un atome d'oxygène, un atome de soufre, un groupe -NH-, un groupe -N(alkyle)- ou un groupe carbonyle,
G³ et G⁴ représentent, indépendamment l'un de l'autre, un atome d'azote ou un groupement 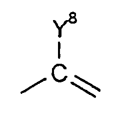 et
Y¹, Y², Y³, Y⁴, Y⁵, Y⁶, Y⁷ et Y⁸ représentent, indépendamment l'un de l'autre, un atome d'hydrogène, un atome d'halogène, un groupe cyano, un groupe nitro; un groupe alkyle, un groupe alcoxy, un groupe alkylamino, un groupe dialkylamino, un groupe alkylthio, un groupe alkylsulfinyle, un groupe alkylsulfonyle ou un groupe cycloalkyle, chacun de ces groupes étant le cas échéant substitué,
R² représente un groupe hydroxyle ou un groupe alcoxy,
G représente un atome d'hydrogène ou, ensemble avec R², un atome d'oxygène doublement lié,
R³ représente un atome d'hydrogène ou un groupe alkyle, un groupe alcényle, un groupe alcynyle ou un groupe cycloalkyle, chacun de ces groupes étant le cas échéant substitué,
R⁴ représente un groupe cycloalkyle, un groupe cycloalcényle, un groupe aryle ou un groupe hétérocyclyle, chacun de ces groupes étant le cas échéant substitué.

2. Composés répondant à la formule (I) selon la revendication 1, dans lesquels
A représente une liaison simple ou un groupe alkylène contenant de 1 à 6 atomes de carbone, portant le cas échéant un ou plusieurs substituants identiques ou différents, les substituants possibles étant sélectionnés de préférence parmi l'énumération ci-après :
un atome d'halogène, un groupe cyano, un groupe nitro, un groupe amino, un groupe hydroxyle, un groupe formyle, un groupe carboxyle, un groupe carbamoyle, un groupe thiocarbamoyle ;
un groupe alcoxy, un groupe alkylthio, un groupe alkylsulfinyle ou un groupe alkylsulfonyle contenant respectivement de 1 à 6 atomes de carbone, chacun de ces groupes étant à chaîne droite ou ramifiée ;
un groupe alcényle ou un groupe alcényloxy contenant respectivement de 2 à 6 atomes de carbone, chacun de ces groupes étant à chaîne droite ou ramifiée ;
un groupe halogénalcoxy, un groupe halogénalkylthio, un groupe halogénalkylsulfinyle ou un groupe halogénalkylsulfonyle contenant respectivement de 1 à 6 atomes de carbone et de 1 à 13 atomes d'halogène identiques ou différents, chacun de ces groupes étant à chaîne droite ou ramifiée ;
un groupe halogénalcényle ou un groupe halogénalcényloxy contenant respectivement de 2 à 6 atomes de carbone et de 1 à 13 atomes d'halogène identiques ou différents, chacun de ces groupes étant à chaîne droite ou ramifiée;
un groupe alkylamino, un groupe dialkylamino, un groupe alkylcarbonyle, un groupe alkylcarbonyloxy, un groupe alcoxycarbonyle, un groupe alkylsulfonyloxy, un groupe hydroximinoalkyle ou un groupe alcoximinoalkyle contenant respectivement de 1 à 6 atomes de carbone dans les fractions alkyle individuelles, chacun de ces groupes étant à chaîne droite ou ramifiée ;
un groupe cycloalkyle contenant de 3 à 6 atomes de carbone ;
ainsi qu'un groupe aryle ou un groupe hétérocyclyle portant, le cas échéant, un ou plusieurs substituants identiques ou différents choisis parmi ceux indiqués ci-après :
un atome d'halogène, un groupe cyano et/ou un groupe alkyle à chaîne droite ou ramifiée contenant de 1 à 4 atomes de carbone
et/ou un groupe halogénalkyle à chaîne droite ou ramifiée contenant de 1 à 4 atomes de carbone et de 1 à 9 atomes d'halogène identiques ou différents
et/ou un groupe alcoxy ou un groupe alkylthio à chaîne droite ou ramifiée contenant de 1 à 4 atomes de carbone
et/ou un groupe halogénalcoxy ou un groupe halogénalkylthio à chaîne droite ou ramifiée contenant de 1 à 4 atomes de carbone et de 1 à 9 atomes d'halogène identiques ou différents
et/ou un groupe alkylène ou un groupe dioxyalkylène contenant respectivement de 1 à 6 atomes de carbone, chacun de ces groupes étant doublement liés et portant le cas échéant un ou plusieurs substituants identiques ou différents halogéno et/ou alkyle à chaîne droite ou ramifiée contenant de 1 à 4 atomes de carbone et/ou halogénalkyle à chaîne droite ou ramifiée contenant de 1 à 4 atomes de carbone et de 1 à 9 atomes d'halogène identiques ou différents,
Q représente un atome d'oxygène ou un atome de soufre,
R¹ représente un groupe phényle ou un groupe naphtyle auquel est condensé un noyau cycloalkyle contenant de 3 à 10 termes cycliques, la fraction cycloalkyle portant le cas échéant, à titre de substituant, de 1 à 4 chaînes alkyle contenant respectivement de 1 à 4 atomes de carbone et la fraction phényle respectivement naphtyle portant le cas échéant les substituants cités pour Y¹ à Y⁸, ou
R¹ représente un groupe benzohétérocyclyle contenant de 3 à 12 termes cycliques dans la fraction hétérocyclyle, ainsi qu'un, deux ou trois hétéroatomes, lié au noyau benzénique et portant le cas échéant un ou plusieurs substituants identiques ou différents, les substituants possibles étant ceux qui ont été cités pour Y¹ à Y⁸, ou
R¹ représente un groupement tricyclique 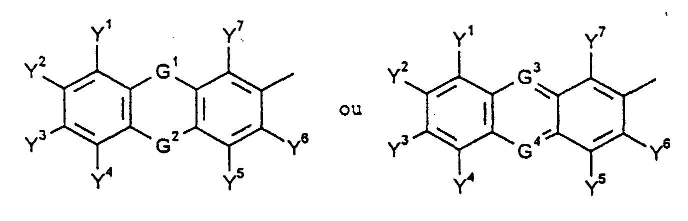 dans lequel
G¹ et G² représentent, indépendamment l'un de l'autre, une liaison simple, un groupe alcanediyle, un groupe alcènediyle, un atome d'oxygène, un atome de soufre, un groupe -NH-, un groupe -N(alkyle)- ou un groupe carbonyle,
G³ et G⁴ représentent, indépendamment l'un de l'autre, un atome d'azote ou un groupement 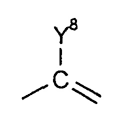 et
Y¹, Y², Y³, Y⁴, Y⁵, Y⁶, Y⁷ et Y⁸ représentent, indépendamment l'un de l'autre, un atome d'hydrogène, un atome d'halogène, un groupe cyano, un groupe nitro ;
un groupe alkyle, un groupe alcoxy, un groupe alkylamino, un groupe dialkylamino, un groupe alkylthio, un groupe alkylsulfinyle ou un groupe alkylsulfonyle, chacun de ces groupes contenant de 1 à 8 atomes de carbone dans les fractions alkyle individuelles ;
un groupe halogénalkyle, un groupe halogénalcoxy, un groupe halogénalkylthio, un groupe halogénalkylsulfinyle ou un groupe halogénalkylsulfonyle, chacun de ces groupes contenant de 1 à 6 atomes de carbone et de 1 à 13 atomes d'halogène identiques ou différents, ou
un groupe cycloalkyle contenant de 3 à 6 atomes de carbone,
R² représente un groupe hydroxyle ou un groupe alcoxy contenant de 1 à 4 atomes de carbone,
G représente un atome d'hydrogène ou, ensemble avec R², un atome d'oxygène doublement lié,
R³ représente un atome d'hydrogène ou un groupe alkyle contenant de 1 à 4 atomes de carbone,
R⁴ représente un groupe aryle, un groupe cycloalkyle ou un groupe cycloalcényle contenant de 3 à 8 atomes de carbone, chacun de ces groupes portant le cas échéant un ou plusieurs substituants identiques ou différents, ou un groupe hétérocyclyle contenant de 3 à 12 termes cycliques, les substituants possibles étant de préférence choisis parmi l'énumération ci-après :
un atome d'halogène, un groupe cyano, un groupe nitro, un groupe amino, un groupe hydroxyle, un groupe formyle, un groupe carboxyle, un groupe carbamoyle, un groupe thiocarbamoyle ;
un groupe alkyle, un groupe alcoxy, un groupe alkylthio, un groupe alkylsulfinyle ou un groupe alkylsulfonyle contenant respectivement de 1 à 6 atomes de carbone, chacun de ces groupes étant à chaîne droite ou ramifiée ;
un groupe alcényle ou un groupe alcényloxy contenant respectivement de 2 à 6 atomes de carbone, chacun de ces groupes étant à chaîne droite ou ramifiée ;
un groupe halogénalkyle, un groupe halogénalcoxy, un groupe halogénalkylthio, un groupe halogénalkylsulfinyle ou un groupe halogénalkylsulfonyle, chacun de ces groupes étant à chaîne droite ou ramifiée et contenant de 1 à 6 atomes de carbone et de 1 à 13 atomes d'halogène identiques ou différents ;
un groupe halogénalcényle ou un groupe halogénalcényloxy contenant respectivement de 2 à 6 atomes de carbone et de 1 à 13 atomes d'halogène identiques ou différents, chacun de ces groupes étant à chaîne droite ou ramifiée;
un groupe alkylamino, un groupe dialkylamino, un groupe alkylcarbonyle, un groupe alkylcarbonyloxy, un groupe alcoxycarbonyle, un groupe alkylsulfonyloxy, un groupe hydroximinoalkyle ou un groupe alcoximinoalkyle contenant respectivement de 1 à 6 atomes de carbone dans les fractions alkyle individuelles, chacun de ces groupes étant à chaîne droite ou ramifiée ;
un groupe alkylène ou un groupe dioxyalkylène contenant respectivement de 1 à 6 atomes de carbone, chacun de ces groupes étant doublement liés et portant le cas échéant un ou plusieurs substituants identiques ou différents halogéno et/ou alkyle à chaîne droite ou ramifiée contenant de 1 à 4 atomes de carbone et/ou halogénalkyle à chaîne droite ou ramifiée contenant de 1 à 4 atomes de carbone et de 1 à 9 atomes d'halogène identiques ou différents,
un groupe cycloalkyle contenant de 3 à 6 atomes de carbone ;
ainsi qu'un groupe aryle, un groupe aryloxy, un groupe arylthio, un groupe arylalkyle, un groupe arylalkyloxy, un groupe arylalkylthio, un groupe hétérocyclyle, un groupe hétérocyclyloxy, un groupe hétérocyclylthio, un groupe hétérocyclylalkyle, un groupe hétérocyclylalkyloxy ou un groupe hétérocyclylalkylthio, chacun de ces groupes portant le cas échéant un ou plusieurs substituants identiques ou différents choisis parmi le groupe comprenant
un atome d'halogène, un groupe cyano et/ou un groupe alkyle à chaîne droite ou ramifiée contenant de 1 à 4 atomes de carbone
et/ou un groupe halogénalkyle à chaîne droite ou ramifiée contenant de 1 à 4 atomes de carbone et de 1 à 9 atomes d'halogène identiques ou différents
et/ou un groupe alcoxy ou un groupe alkylthio à chaîne droite ou ramifiée contenant de 1 à 4 atomes de carbone
et/ou un groupe halogénalcoxy ou un groupe halogénalkylthio à chaîne droite ou ramifiée contenant de 1 à 4 atomes de carbone et de 1 à 9 atomes d'halogène identiques ou différents
et/ou un groupe alkylène ou un groupe dioxyalkylène contenant respectivement de 1 à 6 atomes de carbone, chacun de ces groupes étant doublement liés et portant le cas échéant un ou plusieurs substituants identiques ou différents halogéno et/ou alkyle à chaîne droite ou ramifiée contenant de 1 à 4 atomes de carbone et/ou halogénalkyle à chaîne droite ou ramifiée contenant de 1 à 4 atomes de carbone et de 1 à 9 atomes d'halogène identiques ou différents.

3. Composés répondant à la formule (I) selon la revendication 1, dans lesquels
A représente une liaison simple ou bien un groupe méthylène, un groupe 1,1-éthylène, un groupe 1,2-éthylène, un groupe 1,1-, un groupe 1,2-, un groupe 1,3- ou un groupe 2,2-propylène, un groupe 1,1-, un groupe 1,2-, un groupe 1,3-, un groupe 1,4-, un groupe 2,2-, un groupe 2,3-butylène ou un groupe 1,1-, un groupe 1,2-, un groupe 1,3-(2-méthylpropylène), chacun de ces groupes portant le cas échéant un ou plusieurs substituants identiques ou différents fluoro, chloro, cyano ou méthoxy,
Q représente un atome d'oxygène ou un atome de soufre,
R¹ représente un groupement 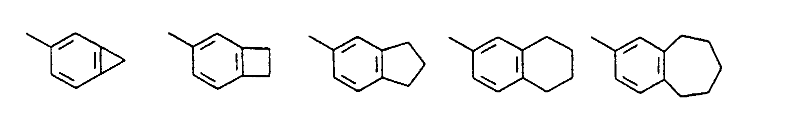 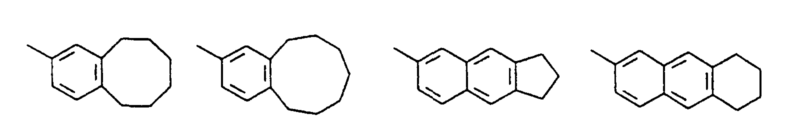 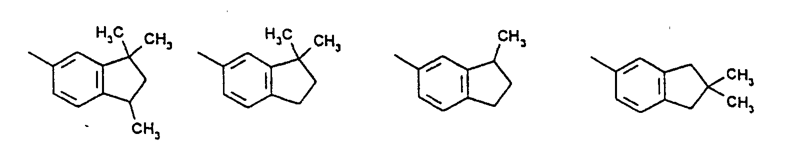 qui porte le cas échéant sur la fraction phényle respectivement naphtyle les substituants cités pour Y¹ à Y⁸, ou
R¹ représente un des groupements ci-après : 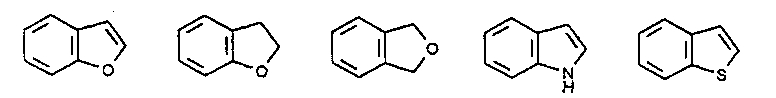 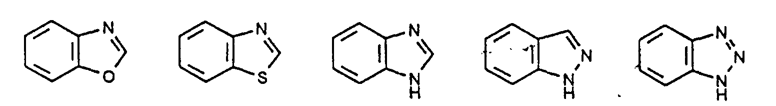 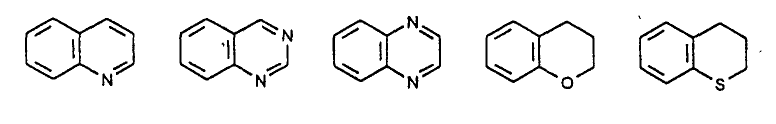 qui sont liés respectivement via un atome de carbone du noyau benzénique respectif et qui peuvent porter respectivement le cas échéant de un à trois substituants identiques ou différents parmi ceux cités pour Y¹ à Y⁸, ou
R¹ représente un groupement tricyclique 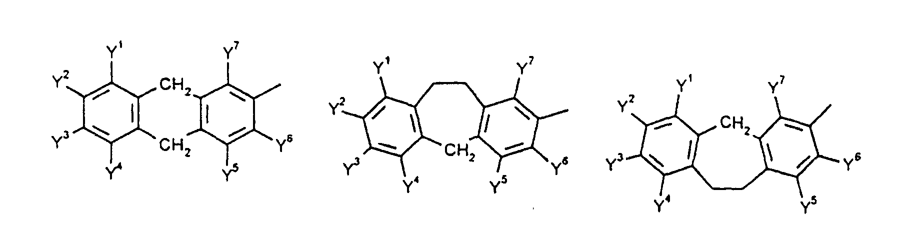 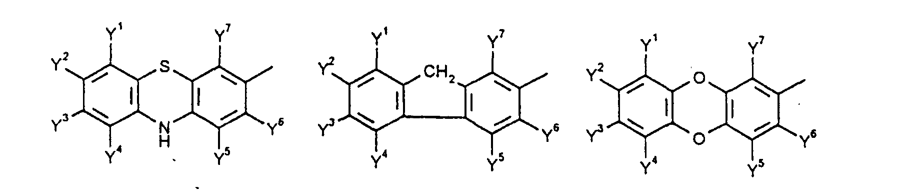 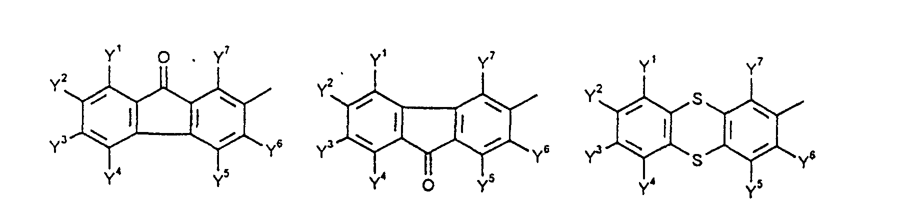 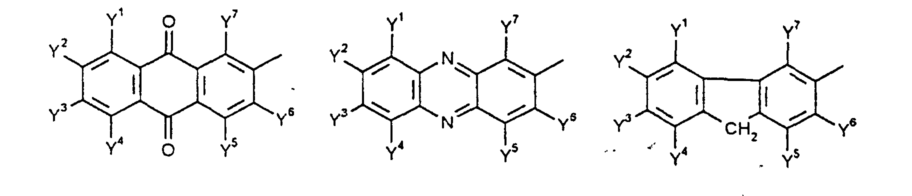 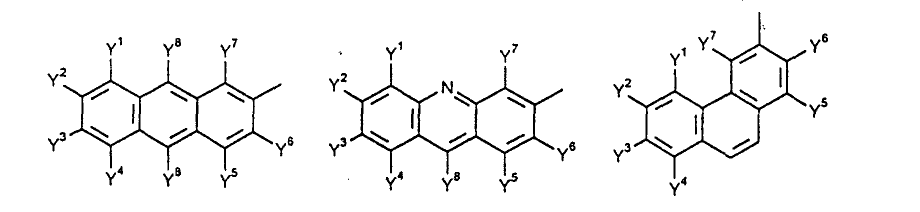 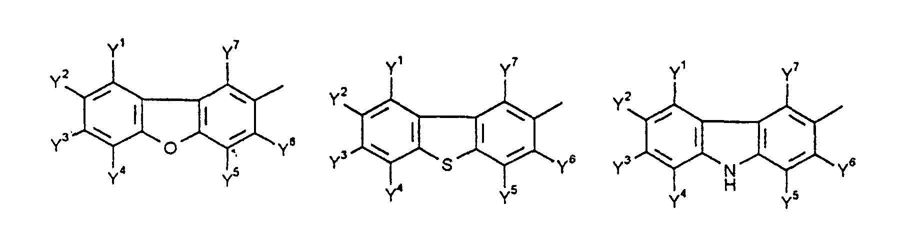 dans lesquels
Y¹, Y², Y³, Y⁴, Y⁵, Y⁶, Y⁷ et Y⁸ représentent, indépendamment l'un de l'autre, un atome d'hydrogène, un atome de fluor, un atome de chlore, un atome de brome, un groupe cyano, un groupe nitro, un groupe méthyle, un groupe éthyle, un groupe n-ou un groupe i-propyle, un groupe n-, un groupe i-, un groupe s- ou un groupe t-butyle, un groupe n-pentyle, un groupe n-hexyle, un groupe n-heptyle, un groupe méthoxy, un groupe éthoxy, un groupe n-ou un groupe i-propoxy, un groupe méthylthio, un groupe éthylthio, un groupe n- ou un groupe i-propylthio, un groupe méthylsulfinyle, un groupe éthylsulfinyle, un groupe méthylsulfonyle ou un groupe éthylsulfonyle, un groupe trifluorométhyle, un groupe trifluoroéthyle, un groupe difluorométhoxy, un groupe trifluorométhoxy, un groupe difluorochlorométhoxy, un groupe trifluoroéthoxy, un groupe difluorométhylthio, un groupe difluorochlorométhylthio, un groupe trifluorométhylthio, un groupe trifluorométhylsulfinyle ou un groupe trifluorométhylsulfonyle, un groupe méthylamino, un groupe éthylamino, un groupe n- ou un groupe i-propylamino, un groupe diméthylamino, un groupe diéthylamino, un groupe cyclopropyle, un groupe cyclobutyle, un groupe cyclopentyle ou un groupe cyclohexyle,
R² représente un groupe hydroxyle, un groupe méthoxy ou un groupe éthoxy,
G représente un atome d'hydrogène ou, ensemble avec R², un atome d'oxygène doublement lié,
R³ représente un atome d'hydrogène ou un groupe méthyle,
R⁴ représente un groupe cyclopropyle, un groupe cyclobutyle, un groupe cyclopentyle ou un groupe cyclohexyle, chacun de ces groupes portant le cas échéant de un à six substituants identiques ou différents, les substituants indiqués ci-dessous étant préférés ;
un groupe phényle, un groupe naphtyle, un groupe furyle, un groupe benzofuranyle, un groupe pyrrolyle, un groupe indolyle, un groupe thiényle, un groupe benzothiényle, un groupe oxazolyle, un groupe isoxazolyle, un groupe thiazolyle, un groupe isothiazolyle, un groupe oxadiazolyle, un groupe thiadiazolyle, un groupe pyridyle, un groupe quinolyle, un groupe pyrimidyle, un groupe pyridazinyle, un groupe pyrazinyle, un groupe oxiranyle, un groupe oxétanyle, un groupe tétrahydrofuryle, un groupe perhydropyranyle, un groupe pyrrolidinyle, un groupe pipéridinyle ou un groupe morpholinyle, chacun de ces groupes portant le cas échéant de un à trois substituants identiques ou différents, les substituants possibles étant sélectionnés de préférence parmi l'énumération ci-après :
un atome de fluor, un atome de chlore, un atome de brome, un groupe cyano, un groupe nitro, un groupe amino, un groupe hydroxyle, un groupe formyle, un groupe carboxyle, un groupe carbamoyle, un groupe thiocarbamoyle, un groupe méthyle, un groupe éthyle, un groupe n- ou un groupe i-propyle, un groupe n-, un groupe i-, un groupe s- ou un groupe t-butyle, un groupe méthoxy, un groupe éthoxy, un groupe n- ou un groupe i-propoxy, un groupe méthylthio, un groupe éthylthio, un groupe n-ou un groupe i-propylthio, un groupe méthylsulfinyle, un groupe éthylsulfinyle, un groupe méthylsulfonyle ou un groupe éthylsulfonyle, un groupe trifluorométhyle, un groupe trifluoroéthyle, un groupe difluorométhoxy, un groupe trifluorométhoxy, un groupe difluorochlorométhoxy, un groupe trifluoroéthoxy, un groupe difluorométhylthio, un groupe difluorochlorométhylthio, un groupe trifluorométhylthio, un groupe trifluorométhylsulfinyle ou un groupe trifluorométhylsulfonyle, un groupe méthylamino, un groupe éthylamino, un groupe n- ou un groupe i-propylamino, un groupe diméthylamino, un groupe diéthylamino, un groupe acétyle, un groupe propionyle, un groupe acétyloxy, un groupe méthoxycarbonyle, un groupe éthoxycarbonyle, un groupe méthylsulfonyloxy, un groupe éthylsulfonyloxy, un groupe hydroximinométhyle, un groupe hydroximinoéthyle, un groupe méthoximinométhyle, un groupe éthoximinométhyle, un groupe méthoximinoéthyle ou un groupe éthoximinoéthyle; un groupe triméthylène (un groupe propane-1,3-diyle), un groupe tétraméthylène (un groupe butane-1,4-diyle), un groupe méthylènedioxy ou un groupe éthylènedioxy, chacun de ces quatre groupes étant doublement liés et portant le cas échéant un ou plusieurs substituants identiques ou différents fluoro, chloro, méthyle, trifluorométhyle, éthyle, n-ou i-propyle,
un groupe cyclopropyle, un groupe cyclobutyle, un groupe cyclopentyle ou un groupe cyclohexyle.

4. Composés répondant à la formule (I) selon la revendication 1, dans lesquels
A représente une liaison simple ou bien un groupe méthylène, un groupe 1,1-éthylène, un groupe 1,2-éthylène, un groupe 1,1-propylène, un groupe 1,2-propylène, un groupe 1,3-propylène ou un groupe 2,2-propylène,
Q représente un atome d'oxygène,
R¹ représente un groupement 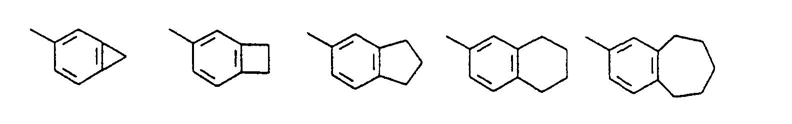 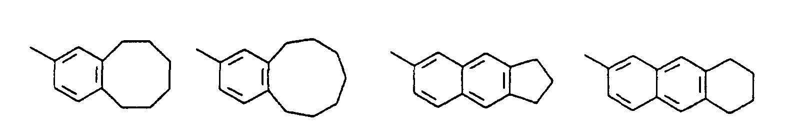 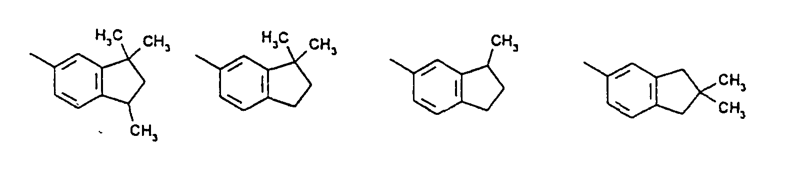 qui porte le cas échéant sur la fraction phényle respectivement naphtyle les substituants cités pour Y¹ à Y⁸, ou
R¹ représente un des groupements ci-après : 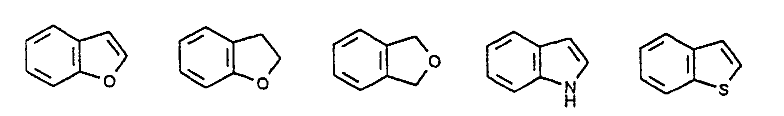 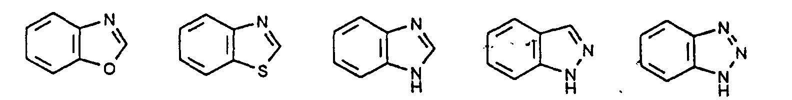 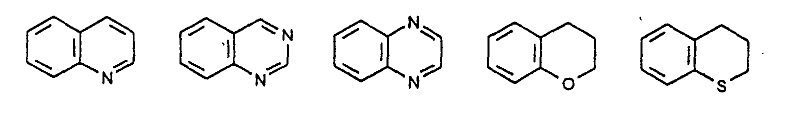 qui sont liés respectivement via un atome de carbone du noyau benzénique respectif et qui peuvent porter respectivement le cas échéant de un à trois substituants identiques ou différents parmi ceux cités pour Y¹ à Y⁸, ou
R¹ représente un groupement tricyclique 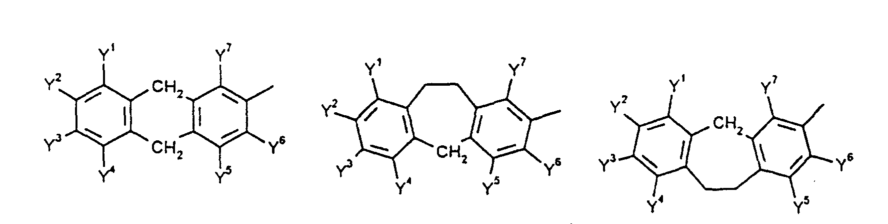 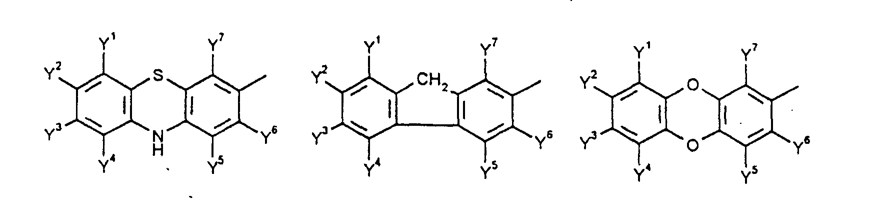 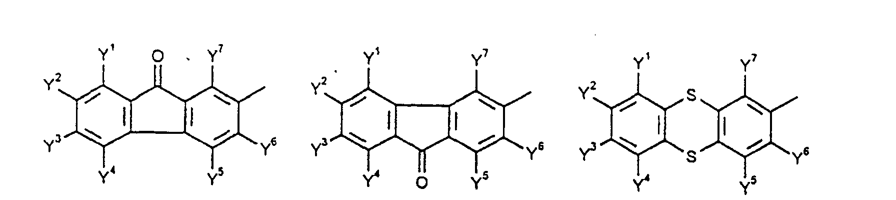 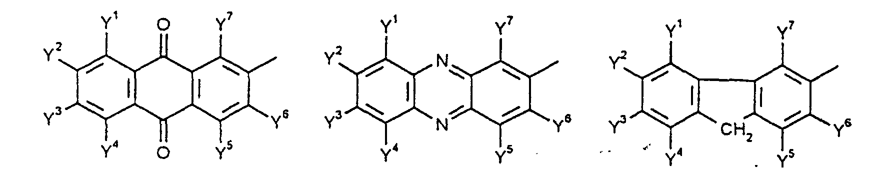 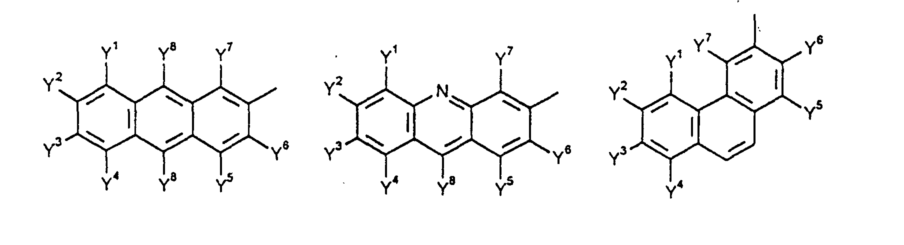 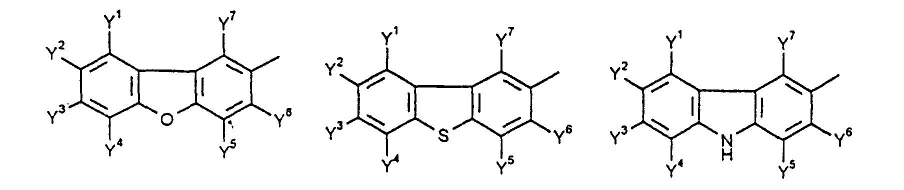 dans lesquels
Y¹, Y², Y³, Y⁴, Y⁵, Y⁶, Y⁷ et Y⁸ représentent, indépendamment l'un de l'autre, un atome d'hydrogène, un atome de fluor, un atome de chlore, un atome de brome, un groupe cyano, un groupe nitro, un groupe méthyle, un groupe éthyle, un groupe n-ou un groupe i-propyle, un groupe n-, un groupe i-, un groupe s- ou un groupe t-butyle, un groupe n-pentyle, un groupe n-hexyle, un groupe n-heptyle, un groupe méthoxy, un groupe éthoxy, un groupe n-ou un groupe i-propoxy, un groupe méthylthio, un groupe éthylthio, un groupe n- ou un groupe i-propylthio, un groupe méthylsulfinyle, un groupe éthylsulfinyle, un groupe méthylsulfonyle ou un groupe éthylsulfonyle, un groupe trifluorométhyle, un groupe trifluoroéthyle, un groupe difluorométhoxy, un groupe trifluorométhoxy, un groupe difluorochlorométhoxy, un groupe trifluoroéthoxy, un groupe difluorométhylthio, un groupe difluorochlorométhylthio, un groupe trifluorométhylthio, un groupe trifluorométhylsulfinyle ou un groupe trifluorométhylsulfonyle, un groupe méthylamino, un groupe éthylamino, un groupe n- ou un groupe i-propylamino, un groupe diméthylamino, un groupe diéthylamino, un groupe cyclopropyle, un groupe cyclobutyle, un groupe cyclopentyle ou un groupe cyclohexyle,
R² représente un groupe hydroxyle, un groupe méthoxy ou un groupe éthoxy,
G représente un atome d'hydrogène ou, ensemble avec R², un atome d'oxygène doublement lié,
R³ représente un atome d'hydrogène ou un groupe méthyle,
R⁴ représente un groupe cyclohexyle ou bien un groupe phényle, un groupe thiényle, un groupe furyle, un groupe benzofuryle, un groupe benzothiényle, un groupe pyridyle, un groupe pyrimidyle, un groupe naphtyle, un groupe quinolyle, chacun de ces groupes portant le cas échéant de un à trois substituants identiques ou différents, les substituants possibles étant choisis de préférence parmi l'énumération ci-après :
un atome de fluor, un atome de chlore, un atome de brome, un groupe cyano, un groupe nitro, un groupe amino, un groupe hydroxyle, un groupe formyle, un groupe carboxyle, un groupe carbamoyle, un groupe thiocarbamoyle, un groupe méthyle, un groupe éthyle, un groupe n- ou un groupe i-propyle, un groupe n-, un groupe i-, un groupe s- ou un groupe t-butyle, un groupe méthoxy, un groupe éthoxy, un groupe n- ou un groupe i-propoxy, un groupe méthylthio, un groupe éthylthio, un groupe n-ou un groupe i-propylthio, un groupe méthylsulfinyle, un groupe éthylsulfinyle, un groupe méthylsulfonyle ou un groupe éthylsulfonyle, un groupe trifluorométhyle, un groupe trifluoroéthyle, un groupe difluorométhoxy, un groupe trifluorométhoxy, un groupe difluorochlorométhoxy, un groupe trifluoroéthoxy, un groupe difluorométhylthio, un groupe difluorochlorométhylthio, un groupe trifluorométhylthio, un groupe trifluorométhylsulfinyle ou un groupe trifluorométhylsulfonyle, un groupe méthylamino, un groupe éthylamino, un groupe n- ou un groupe i-propylamino, un groupe diméthylamino, un groupe diéthylamino, un groupe acétyle, un groupe propionyle, un groupe acétyloxy, un groupe méthoxycarbonyle, un groupe éthoxycarbonyle, un groupe méthylsulfonyloxy, un groupe éthylsulfonyloxy, un groupe hydroximinométhyle, un groupe hydroximinoéthyle, un groupe méthoximinométhyle, un groupe éthoximinométhyle, un groupe méthoximinoéthyle ou un groupe éthoximinoéthyle;
un groupe triméthylène (un groupe propane-1,3-diyle), un groupe tétraméthylène (un groupe butane-1,4-diyle), un groupe méthylènedioxy ou un groupe éthylènedioxy, chacun de ces groupes étant doublement liés et portant le cas échéant un ou plusieurs substituants identiques ou différents fluoro, chloro, méthyle, trifluorométhyle, éthyle, n- ou i-propyle.

5. Composés répondant à la formule (I) selon la revendication 1, dans lesquels
R² représente un groupe hydroxyle.

6. Agents de lutte contre les parasites, **caractérisé en** une teneur en au moins un composé répondant à la formule (I) selon la revendication 1.

7. Procédé pour lutter contre les parasites, **caractérisé en ce qu'**on laisse agir des composés répondant à la formule (I) selon la revendication 1 sur les parasites et/ou sur leur biotope.

8. Procédé pour la préparation de composés répondant à la formule (I), **caractérisé en ce que**
a) on fait réagir des esters glyoxyliques répondant à la formule (II) 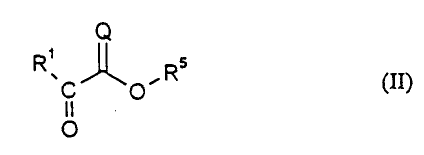 dans laquelle
Q et R¹ ont les significations indiquées ci-dessus et
R⁵ représente un groupe alkyle,
avec une amine répondant à la formule (III), 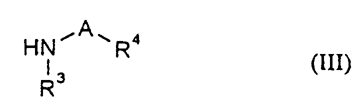 dans laquelle
A, R³ et R⁴ ont les significations indiquées ci-dessus,
le cas échéant en présence d'un diluant et le cas échéant en présence d'une base, ou bien
b) on fait réagir des amides d'acide glyoxylique répondant à la formule (I-b) 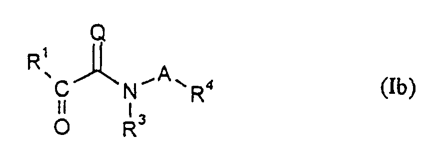 dans laquelle
A, Q, R¹, R³ et R⁴ ont les significations indiquées ci-dessus,
avec de l'hydrogène ou avec un métal non noble, le cas échéant en présence d'un catalyseur et le cas échéant en présence d'un adjuvant réactionnel supplémentaire.

9. Utilisation de composés répondant à la formule (I) selon les revendications 1 à 8 pour lutter contre les parasites.

10. Procédé pour la préparation d'agents de lutte contre les parasites, **caractérisé en ce qu'**on mélange des composés répondant à la formule (I) selon les revendications 1 à 8 avec des diluants et/ou avec des agents tensioactifs.
